# EUROPEAN PATENT APPLICATION

(11) **EP 3 950 881 A1**
(43) Date of publication of application: **09.02.2022**
(21) Application number: 20781856.8
(22) Date of filing: 16.03.2020
(51) Int. Cl.: C09K 11/06, H05B 33/10, H01L 51/50

(54) **LIGHT-EMITTING ELEMENT AND METHOD FOR PRODUCING SAME, AND COMPOSITION FOR LIGHT-EMITTING ELEMENT AND METHOD FOR PRODUCING SAME**

(30) Priority: 29.03.2019 JP 2019066194
(71) Applicant: SUMITOMO CHEMICAL COMPANY LIMITED, Chuo-ku Tokyo 104-8260 (JP)
(72) Inventor: SASADA, Toshiaki, Tsukuba-shi, Ibaraki 300-3294 (JP); MATSUMOTO, Ryuji, Ishikawa 923-1201 (JP)
(74) Representative: J A Kemp LLP
(86) International application number: PCT/JP2020/011384
(87) International publication number: WO 2020/203210

(57) **Abstract**

To provide a composition which is useful for producing a light emitting device excellent in light emission efficiency, and a light emitting device comprising the composition.

A light emitting device comprising an anode, a cathode, and an organic layer disposed between the anode and the cathode and containing a composition for light emitting device, wherein the composition for light emitting device contains a thermally activated delayed fluorescent compound (A), a boron atom, and a compound (B) having a condensed hetero ring skeleton (b), |EB-AA| is 0.60 eV or less, ΔE_{ST}(A) of the compound (A) is 0.50 eV or less, and ΔE_{ST}(B) of the compound (B) is 0.50 eV or less.

## Description

### Technical Field

The present invention relates to a light emitting device and a production method thereof. Further, the present invention relates to a composition for light emitting layer and a production method thereof.

### Background Art

Light emitting devices such as an organic electroluminescent device and the like can be suitably used for, for example, display and illumination. As a light emitting material used for a light emitting layer of a light emitting device, for example, Patent Document 1 suggests a composition containing only one type of thermally activated delayed fluorescent compound. Patent Document 2 suggests a composition containing two types of thermally activated delayed fluorescent compounds. The two types of thermally activated delayed fluorescent compounds contained in this composition are compounds not having a condensed hetero ring skeleton (b) described later.

### Prior Art Document

### Patent Document

Patent Document 1: International Publication WO2018/062278
Patent Document 2: International Publication WO2015/135625

### Summary of the Invention

### Problem to be Solved by the Invention

However, light emitting devices fabricated using the above-described composition were not necessarily sufficient in light emission efficiency.

Then, the present invention has an object of providing a composition which is useful for producing a light emitting device excellent in light emission efficiency, and a light emitting device comprising the composition.

### Means for Solving the Problem

The present inventors have intensively studied to solve the above-described problem and resultantly found that in a composition for light emitting device containing a specific thermally activated delayed fluorescent compound (A) and a specific compound (B), if a difference between the energy level of the lowest triplet excited state and the energy level of the lowest single excited state of each compound satisfies a specific condition and a difference (EB-AA) between the energy value (EB) of the maximum peak of the emission spectrum at 25°C of the compound (B) and the energy value (AA) of a peak at the lowest energy side of the absorption spectrum at 25°C of the compound (A) satisfies a specific condition, then, a light emitting device excellent in light emission efficiency is formed, leading to completion of the present invention.

That is, the present invention provides the following [1] to [28].
[1] A light emitting device comprising
   an anode,
   a cathode, and
   an organic layer disposed between the above-described anode and the above-described cathode and containing a composition for light emitting device, wherein
   the above-described composition for light emitting device contains
   a thermally activated delayed fluorescent compound (A), and
   a compound (B) having a condensed hetero ring skeleton (b) containing a boron atom and at least one selected from the group consisting of an oxygen atom, a sulfur atom, a selenium atom, an sp³ carbon atom and a nitrogen atom in the ring,
   the above-described thermally activated delayed fluorescent compound (A) is a compound not having the above-described condensed hetero ring skeleton (b),
   the absolute value (|EB-AA|) of a difference between the energy value (EB) of the maximum peak of the emission spectrum at 25°C of the above-described compound (B) and the energy value (AA) of a peak at the lowest energy side of the absorption spectrum at 25°C of the above-described compound (A) is 0.60 eV or less,
   the absolute value ΔE_{ST}(A) of a difference between the energy level of the lowest triplet excited state and the energy level of the lowest singlet excited state of the above-described compound (A) is 0.50 eV or less, and
   the absolute value ΔE_{ST}(B) of a difference between the energy level of the lowest triplet excited state and the energy level of the lowest singlet excited state of the above-described compound (B) is 0.50 eV or less.
[2] The light emitting device according to [1], wherein the above-described ΔE_{ST}(B) is larger than the above-described ΔE_{ST}(A).
[3] The light emitting device according to [1] or [2], wherein the above-described condensed hetero ring skeleton (b) contains a boron atom and at least one selected from the group consisting of an oxygen atom, a sulfur atom and a nitrogen atom in the ring.
[4] The light emitting device according to any one of [1] to [3], wherein the above-described compound (B) is a compound represented by the formula (1-1), a compound represented by the formula (1-2) or a compound represented by the formula (1-3): 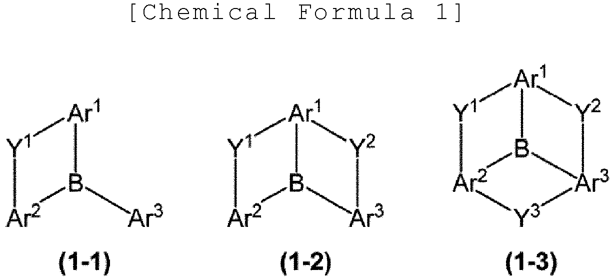 [wherein,
   Ar¹, Ar² and Ar³ each independently represent an aromatic hydrocarbon group or a hetero ring group, and these groups optionally have a substituent. When a plurality of the substituents are present, they may be the same or different and may be combined together to form a ring together with atoms to which they are attached.
   Y¹ represents an oxygen atom, a sulfur atom, a selenium atom, a group represented by -N(Ry)-, an alkylene group or a cycloalkylene group, and these groups optionally have a substituent. When a plurality of the substituents are present, they may be the same or different and may be combined together to form a ring together with atoms to which they are attached.
   Y² and Y³ each independently represent a single bond, an oxygen atom, a sulfur atom, a selenium atom, a group represented by -N(Ry)-, an alkylene group or a cycloalkylene group, and these groups optionally have a substituent. When a plurality of the substituents are present, they may be the same or different and may be combined together to form a ring together with atoms to which they are attached. Ry represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of Ry are present, they may be the same or different. Ry may be bonded directly or via a connecting group to Ar¹, Ar² or Ar³.].
[5] The light emitting device according to [4], wherein the above-described Y¹, the above-described Y² and the above-described Y³ are each an oxygen atom, a sulfur atom or a group represented by - N(Ry)-.
[6] The light emitting device according to any one of [1] to [5], wherein the above-described compound (A) is a compound represented by the formula (T-1): 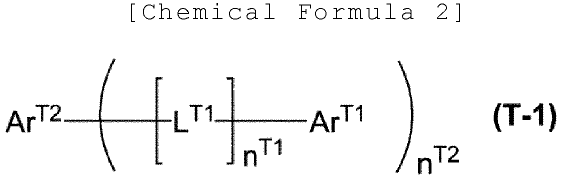 [wherein,
   n^{T1} represents an integer of 0 or more. When a plurality of n^{T1} are present, they may be the same or different.
   n^{T2} represents an integer of 1 or more. n^{T2} is 2, when Ar^{T2} is a group represented by -C(=O)-, a group represented by -S(=O)- or a group represented by -S(=O)₂-.
   Ar^{T1} represents a substituted amino group or a monovalent hetero ring group, and these groups optionally have a substituent. When a plurality of the substituents are present, they may be the same or different and may be combined together to form a ring together with atoms to which they are attached. When a plurality of Ar^{T1} are present, they may be the same or different.

The monovalent hetero ring group represented by Ar^{T1} is a monovalent hetero ring group containing a nitrogen atom not forming a double bond in the ring and not containing a group represented by =N-, a group represented by -C(=O)-, a group represented by -S(=O)- and a group represented by -S(=O)₂- in the ring.

L^{T1} represents an alkylene group, a cycloalkylene group, an arylene group, a divalent hetero ring group, an oxygen atom or a sulfur atom, and these groups optionally have a substituent. When a plurality of the substituents are present, they may be the same or different and may be combined together to form a ring together with atoms to which they are attached. When a plurality of L^{T1} are present, they may be the same or different.

Ar^{T2} is a group represented by -C(=O)-, a group represented by -S(=O)-, a group represented by - S(=O)₂-, an aromatic hydrocarbon group having an electron-attracting group, an aromatic hydrocarbon group containing a group represented by -C(=O)- in the ring or a hetero ring group containing at least one group selected from the group consisting of a group represented by =N-, a group represented by - C(=O)-, a group represented by -S(=O)- and a group represented by -S(=O)₂- in the ring, and these groups optionally have a substituent. When a plurality of the substituents are present, they may be the same or different and may be combined together to form a ring together with atoms to which they are attached.].

[7] The light emitting device according to any one of [1] to [6], wherein the above-described composition for light emitting device further contains a host material.

[8] The light emitting device according to [7], wherein the above-described host material contains a compound represented by the formula (H-1): 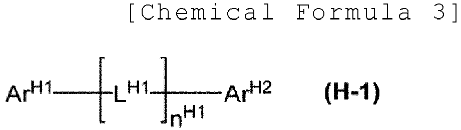 [wherein,

Ar^{H1} and Ar^{H2} each independently represent an aryl group, a monovalent hetero ring group or a substituted amino group, and these groups optionally have a substituent. When a plurality of the substituents are present, they may be the same or different and may be combined together to form a ring together with atoms to which they are attached.

n^{H1} represents an integer of 0 or more.

L^{H1} represents an arylene group, a divalent hetero ring group, an alkylene group or a cycloalkylene group, and these groups optionally have a substituent. When a plurality of the substituents are present, they may be the same or different and may be combined together to form a ring together with atoms to which they are attached. When a plurality of L^{H1} are present, they may be the same or different.].

[9] The light emitting device according to [7] or [8], wherein the absolute value (|EH-AB|) of a difference between the energy value (EH) of the maximum peak of the emission spectrum at 25°C of the above-described host material and the energy value (AB) of a peak at the lowest energy side of the absorption spectrum at 25°C of the above-described compound (B) is 0.60 eV or less.

[10] The light emitting device according to any one of [1] to [9], wherein the above-described composition for light emitting device further contains at least one selected from the group consisting of a hole transporting material, a hole injection material, an electron transporting material, an electron injection material, a light emitting material, an antioxidant and a solvent.

[11] A composition for light emitting device comprising
a thermally activated delayed fluorescent compound (A) and
a compound (B) having a condensed hetero ring skeleton (b) containing a boron atom and at least one selected from the group consisting of an oxygen atom, a sulfur atom, a selenium atom, an sp³ carbon atom and a nitrogen atom in the ring, wherein
the above-described thermally activated delayed fluorescent compound (A) is a compound not having the above-described condensed hetero ring skeleton (b),
the absolute value (|EB-AA|) of a difference between the energy value (EB) of the maximum peak of the emission spectrum at 25°C of the above-described compound (B) and the energy value (AA) of a peak at the lowest energy side of the absorption spectrum at 25°C of the above-described compound (A) is 0.60 eV or less,
the absolute value ΔE_{ST}(A) of a difference between the energy level of the lowest triplet excited state and the energy level of the lowest singlet excited state of the above-described compound (A) is 0.50 eV or less, and
the absolute value ΔE_{ST}(B) of a difference between the energy level of the lowest triplet excited state and the energy level of the lowest singlet excited state of the above-described compound (B) is 0.50 eV or less.

[12] The composition for light emitting device according to [11], further comprising a host material.

[13] The composition for light emitting device according to [12], wherein the above-described host material contains a compound represented by the formula (H-1): 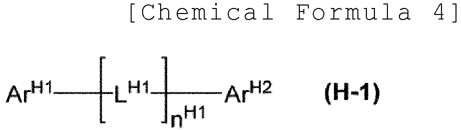 [wherein,

Ar^{H1} and Ar^{H2} each independently represent an aryl group, a monovalent hetero ring group or a substituted amino group, and these groups optionally have a substituent. When a plurality of the substituents are present, they may be the same or different and may be combined together to form a ring together with atoms to which they are attached.

n^{H1} represents an integer of 0 or more.

L^{H1} represents an arylene group, a divalent hetero ring group, an alkylene group or a cycloalkylene group, and these groups optionally have a substituent. When a plurality of the substituents are present, they may be the same or different and may be combined together to form a ring together with atoms to which they are attached. When a plurality of L^{H1} are present, they may be the same or different.].

[14] The composition for light emitting device according to [12] or [13], wherein the absolute value (|EH-AB|) of a difference between the energy value (EH) of the maximum peak of the emission spectrum at 25°C of the above-described host material and the energy value (AB) of a peak at the lowest energy side of the absorption spectrum at 25°C of the above-described compound (B) is 0.60 eV or less.

[15] The composition for light emitting device according to any one of [11] to [14], wherein the above-described composition for light emitting device further contains at least one selected from the group consisting of a hole transporting material, a hole injection material, an electron transporting material, an electron injection material, a light emitting material, an antioxidant and a solvent.

[16] A method for producing a composition for light emitting device, comprising
a preparation step of preparing a thermally activated delayed fluorescent compound (A) in which the absolute value ΔE_{ST}(A) of a difference between the energy level of the lowest triplet excited state and the energy level of the lowest singlet excited state is 0.50 eV or less,
a sorting step of sorting a compound (B) which is a compound having a condensed hetero ring skeleton (b) containing a boron atom and at least one selected from the group consisting of an oxygen atom, a sulfur atom, a selenium atom, an sp³ carbon atom and a nitrogen atom in the ring and in which the absolute value ΔE_{ST}(B) of a difference between the energy level of the lowest triplet excited state and the energy level of the lowest singlet excited state is 0.50 eV or less and the energy value (EB) of the maximum peak of the emission spectrum at 25°C shows a value with which the absolute value (|EB-AA|) of a difference from the energy value (AA) of a peak at the lowest energy side of the absorption spectrum at 25°C of the above-described compound (A) is 0.60 eV or less, and
a production step of mixing the compound (A) prepared in the above-described preparation step and the compound (B) sorted in the above-described sorting step to obtain a composition for light emitting device, wherein
the above-described thermally activated delayed fluorescent compound (A) is a compound not having the above-described condensed hetero ring skeleton (b).

[17] The production method according to [16], wherein the above-described sorting step includes a step of determining the energy value (EB) of the maximum peak of the emission spectrum at 25°C of the above-described compound (B) and the energy value (AA) of a peak at the lowest energy side of the absorption spectrum at 25°C of the above-described compound (A) and calculating the absolute value (|EB-AA|) of a difference thereof.

[18] The production method according to [16] or [17], wherein the above-described production step is a step of mixing the above-described compound (A) prepared in the above-described preparation step, the above-described compound (B) sorted in the above-described sorting step, and a host material.

[19] The production method according to [18], wherein
the above-described sorting step further includes a step of sorting the above-described compound (B) such that the absolute value (|EH-AB|) of a difference between the energy value (EH) of the maximum peak of the emission spectrum at 25°C of the above-described host material and the energy value (AB) of a peak at the lowest energy side of the absorption spectrum at 25°C of the above-described compound (B) is 0.60 eV or less.

[20] The production method according to [16] or [17], further comprising a host material sorting step of sorting the host material such that the absolute value (|EH-AB|) of a difference between the energy value (EH) of the maximum peak of the emission spectrum at 25°C of the host material and the energy value (AB) of a peak at the lowest energy side of the absorption spectrum at 25°C of the above-described compound (B) sorted in the above-described sorting step is 0.60 eV or less, wherein
the above-described production step is a step of mixing the above-described compound (A) prepared in the above-described preparation step, the above-described compound (B) sorted in the above-described sorting step, and the above-described host material sorted in the above-described host material sorting step.

[21] The production method according to any one of [18] to [20], wherein the above-described host material contains a compound represented by the formula (H-1): 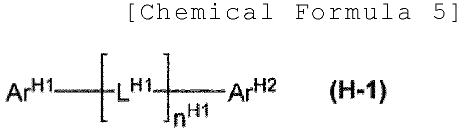 [wherein,

Ar^{H1} and Ar^{H2} each independently represent an aryl group, a monovalent hetero ring group or a substituted amino group, and these groups optionally have a substituent. When a plurality of the substituents are present, they may be the same or different and may be combined together to form a ring together with atoms to which they are attached.

n^{H1} represents an integer of 0 or more.

L^{H1} represents an arylene group, a divalent hetero ring group, an alkylene group or a cycloalkylene group, and these groups optionally have a substituent. When a plurality of the substituents are present, they may be the same or different and may be combined together to form a ring together with atoms to which they are attached. When a plurality of L^{H1} are present, they may be the same or different.].

[22] A method for producing a composition for light emitting device, comprising
a preparation step of preparing a compound (B) in which the absolute value ΔE_{ST}(B) of a difference between the energy level of the lowest triplet excited state and the energy level of the lowest singlet excited state is 0.50 eV or less, and having a condensed hetero ring skeleton (b) containing a boron atom and at least one selected from the group consisting of an oxygen atom, a sulfur atom, a selenium atom, an sp³ carbon atom and a nitrogen atom in the ring,
a sorting step of sorting a thermally activated delayed fluorescent compound (A) in which the absolute value ΔE_{ST}(A) of a difference between the energy level of the lowest triplet excited state and the energy level of the lowest singlet excited state is 0.50 eV or less, and, the energy value (AA) of a peak at the lowest energy side of the absorption spectrum at 25°C shows a value with which the absolute value (|EB-AA|) of a difference from the energy value (EB) of the maximum peak of the emission spectrum at 25°C of the above-described compound (B) is 0.60 eV or less, and
a production step of mixing the compound (B) prepared in the above-described preparation step and the above-described compound (A) sorted in the above-described sorting step to obtain a composition for light emitting device, wherein
the above-described thermally activated delayed fluorescent compound (A) is a compound not having the above-described condensed hetero ring skeleton (b).

[23] The production method according to [22], wherein the above-described sorting step includes a step of determining the energy value (EB) of the maximum peak of the emission spectrum at 25°C of the above-described compound (B) and the energy value (AA) of a peak at the lowest energy side of the absorption spectrum at 25°C of the above-described compound (A) and calculating the absolute value (|EB-AA|) of a difference thereof.

[24] The production method according to [22] or [23], wherein the above-described production step is a step of mixing the above-described compound (B) prepared in the above-described preparation step, the above-described compound (A) sorted in the above-described sorting step, and a host material.

[25] The production method according to [24], further comprising a host material preparation step of preparing a host material, wherein
the above-described preparation step is a step of preparing a compound (B) in which the absolute value (|EH-AB|) of a difference between the energy value (EH) of the maximum peak of the emission spectrum at 25°C of the above-described host material and the energy value (AB) of a peak at the lowest energy side of the absorption spectrum at 25°C of the above-described compound (B) is 0.60 eV or less.

[26] The production method according to [22] or [23], further comprising a host material sorting step of sorting a host material such that the absolute value (|EH-AB|) of a difference between the energy value (EH) of the maximum peak of the emission spectrum at 25°C of the host material and the energy value (AB) of a peak at the lowest energy side of the absorption spectrum at 25°C of the above-described compound (B) prepared in the above-described preparation step is 0.60 eV or less, wherein
the above-described production step is a step of mixing the above-described compound (B) prepared in the above-described preparation step, the above-described compound (A) sorted in the above-described sorting step, and the above-described host material sorted in the above-described host material sorting step.

[27] The production method according to any one of [24] to [26], wherein the above-described host material contains a compound represented by the formula (H-1): 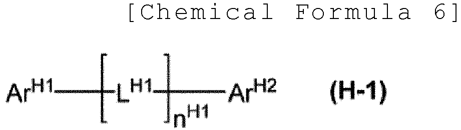 [wherein,
Ar^{H1} and Ar^{H2} each independently represent an aryl group, a monovalent hetero ring group or a substituted amino group, and these groups optionally have a substituent. When a plurality of the substituents are present, they may be the same or different and may be combined together to form a ring together with atoms to which they are attached.
n^{H1} represents an integer of 0 or more.
L^{H1} represents an arylene group, a divalent hetero ring group, an alkylene group or a cycloalkylene group, and these groups optionally have a substituent. When a plurality of the substituents are present, they may be the same or different and may be combined together to form a ring together with atoms to which they are attached. When a plurality of L^{H1} are present, they may be the same or different.].

[28] A method for producing a light emitting device having an anode, a cathode, and an organic layer disposed between the above-described anode and the above-described cathode, comprising
a step of producing a composition for light emitting device by the production method as described in any one of [16] to [27], and a step of forming the above-described organic layer using the above-described composition for light emitting device produced in the above-described step.

### Effect of the Invention

According to the present invention, it is possible to provide a composition which is useful for producing a light emitting device excellent in light emission efficiency, and a production method thereof. Further, according to the present invention, it is possible to provide a light emitting device comprising the composition, and a production method thereof.

### Modes for Carrying Out the Invention

Suitable embodiments of the present invention will be illustrated in detail below.

### <Explanation of common terms>

Terms commonly used in the present specification have the following meanings unless otherwise stated.

### "Room temperature" denotes 25°C.

Me represents a methyl group, Et represents an ethyl group, Bu represents a butyl group, i-Pr represents an isopropyl group, and t-Bu represents a tert-butyl group.

A hydrogen atom may be a heavy hydrogen atom or a light hydrogen atom.

"The low molecular weight compound" means a compound having no molecular weight distribution and having a molecular weight of 1×10⁴ or less.

"The polymer compound" means a polymer having molecular weight distribution and having a polystyrene-equivalent number-average molecular weight of 1×10³ or more (for example, 1×10³ to 1×10⁸).

"The constitutional unit" means a unit occurring once or more times in the polymer compound.

The polymer compound may be any of a block copolymer, a random copolymer, an alternating copolymer and a graft copolymer, and may also be another form.

The end group of the polymer compound is preferably a stable group since if a polymerization active group remains intact there, there is a possibility of a decrease in a light emitting property or luminance life when the polymer compound is used for fabrication of a light emitting device. The end group of the polymer compound is preferably a group conjugatively bonded to the main chain and includes, for example, groups bonding to an aryl group or a monovalent hetero ring group linking to the main chain of the polymer compound via a carbon-carbon bond.

"The alkyl group" may be any of linear or branched. The number of carbon atoms of the linear alkyl group, not including the number of carbon atoms of the substituent, is usually 1 to 50, preferably 1 to 20, and more preferably 1 to 10. The number of carbon atoms of the branched alkyl group, not including the number of carbon atoms of the substituent, is usually 3 to 50, preferably 3 to 20, and more preferably 4 to 10.

The alkyl group optionally has a substituent. The alkyl group includes, for example, a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group. Further, the alkyl group may also be a group obtained by substituting a part or all of hydrogen atoms in these groups with a cycloalkyl group, an alkoxy group, a cycloalkoxy group, an aryl group, a fluorine atom or the like. Such an alkyl group includes, for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, 3-(3,5-di-hexylphenyl)propyl group and a 6-ethyloxyhexyl group.

The number of carbon atoms of "the cycloalkyl group", not including the number of carbon atoms of the substituent, is usually 3 to 50, and preferably 4 to 10. The cycloalkyl group optionally has a substituent. The cycloalkyl group includes, for example, a cyclohexyl group and a methylcyclohexyl group.

The number of carbon atoms of "the alkylene group", not including the number of carbon atoms of the substituent, is usually 1 to 20, preferably 1 to 15, and more preferably 1 to 10. The alkylene group optionally has a substituent. The alkylene group includes, for example, a methylene group, an ethylene group, a propylene group, a butylene group, a hexylene group and an octylene group.

The number of carbon atoms of "the cycloalkylene group", not including the number of carbon atoms of the substituent, is usually 3 to 20, and preferably 4 to 10. The cycloalkylene group optionally has a substituent. The cycloalkylene group includes, for example, a cyclohexylene group.

"The aromatic hydrocarbon group" means a group obtained by removing from an aromatic hydrocarbon one or more hydrogen atoms bonding directly to atoms constituting the ring. The group obtained by removing from an aromatic hydrocarbon one hydrogen atom bonding directly to an atom constituting the ring is referred to also as "an aryl group". The group obtained by removing from an aromatic hydrocarbon two hydrogen atoms bonding directly to atoms constituting the ring is referred to also as "an arylene group".

The number of carbon atoms of the aromatic hydrocarbon group, not including the number of carbon atoms of the substituent, is usually 6 to 60, preferably 6 to 40, and more preferably 6 to 20.

"The aromatic hydrocarbon group" includes, for example, groups obtained by removing from a monocyclic aromatic hydrocarbon (including, for example, benzene) or a polycyclic aromatic hydrocarbon (including, for example, dicyclic aromatic hydrocarbons such as naphthalene, indene, naphthoquinone, indenone, tetralone and the like; tricyclic aromatic hydrocarbons such as anthracene, phenanthrene, dihydrophenanthrene, fluorene, anthraquinone, phenanthoquinone, fluorenone and the like; tetracyclic aromatic hydrocarbons such as benzoanthracene, benzophenanthrene, benzofluorene, pyrene, fluoranthene and the like; pentacyclic aromatic hydrocarbons such as dibenzoanthracene, dibenzophenanthrene, dibenzofluorene, perylene, benzofluoranthene and the like; hexacyclic aromatic hydrocarbons such as spirobifluorene and the like; and, heptacyclic aromatic hydrocarbons such as benzospirobifluorene, acenaphthofluoranthene and the like) one or more hydrogen atoms bonding directly to atoms constituting the ring. The aromatic hydrocarbon group includes groups obtained by bonding a plurality of these groups. The aromatic hydrocarbon group optionally has a substituent.

"The alkoxy group" may be any of linear or branched. The number of carbon atoms of the linear alkoxy group, not including the number of carbon atoms of the substituent, is usually 1 to 40, and preferably 1 to 10. The number of carbon atoms of the branched alkoxy group, not including the number of carbon atoms of the substituent, is usually 3 to 40, and preferably 4 to 10.

The alkoxy group optionally has a substituent. The alkoxy group includes, for example, a methoxy group, an ethoxy group, an isopropyloxy group, a butyloxy group, a hexyloxy group, a 2-ethylhexyloxy group, a 3,7-dimethyloctyloxy group and a lauryloxy group.

The number of carbon atoms of "the cycloalkoxy group", not including the number of carbon atoms of the substituent, is usually 3 to 40, and preferably 4 to 10. The cycloalkoxy group optionally has a substituent. The cycloalkoxy group includes, for example, a cyclohexyloxy group.

The number of carbon atoms of "the aryloxy group", not including the number of carbon atoms of the substituent, is usually 6 to 60, preferably 6 to 40, and more preferably 6 to 20. The aryloxy group optionally has a substituent. The aryloxy group includes, for example, a phenoxy group, a naphthyloxy group, an anthracenyloxy group and a pyrenyloxy group.

"The hetero ring group" means a group obtained by removing from a heterocyclic compound one or more hydrogen atoms bonding directly to atoms constituting the ring. Of the hetero ring groups, "an aromatic hetero ring group" which is a group obtained by removing from an aromatic heterocyclic compound one or more hydrogen atoms bonding directly to atoms constituting the ring is preferable. The group obtained by removing from a heterocyclic compound p hydrogen atoms bonding directly to atoms constituting the ring (p represents an integer of 1 or more) is referred to also as "p-valent hetero ring group". The group obtained by removing from an aromatic heterocyclic compound p hydrogen atoms bonding directly to atoms constituting the ring is referred to also as "p-valent aromatic hetero ring group".

"The aromatic heterocyclic compound" includes, for example, compounds in which the hetero ring itself shows aromaticity such as azole, thiophene, furan, pyridine, diazabenzene, triazine, azanaphthalene, diazanaphthalene, carbazole and the like, and, compounds in which an aromatic ring is condensed to a hetero ring even if the hetero ring itself shows no aromaticity such as phenoxazine, phenothiazine, benzopyran and the like.

The number of carbon atoms of the hetero ring group, not including the number of carbon atoms of the substituent, is usually 1 to 60, preferably 2 to 40, and more preferably 3 to 20. The number of hetero atoms of the aromatic hetero ring group, not including the number of hetero atoms of the substituent, is usually 1 to 30, preferably 1 to 10, more preferably 1 to 5, and further preferably 1 to 3.

The hetero ring group includes, for example, groups obtained by removing from a monocyclic heterocyclic compound (including, for example, furan, thiophene, oxadiazole, thiadiazole, pyrrole, diazole, triazole, tetrazole, pyridine, diazabenzene and triazine) or a polycyclic heterocyclic compound (including, for example, dicyclic heterocyclic compounds such as azanaphthalene, diazanaphthalene, benzofuran, benzothiophene, indole, azaindole, diazaindole, benzodiazole, benzothiadiazole, benzotriazole, benzothiophene dioxide, benzothiophene oxide, benzopyranone and the like; tricyclic heterocyclic compounds such as dibenzofuran, dibenzothiophene, dibenzothiophene dioxide, dibenzothiophene oxide, dibenzopyranone, dibenzoborole, dibenzosilole, dibenzophosphole, dibenzoselenophene, carbazole, azacarbazole, diazacarbazole, phenoxazine, phenothiazine, 9,10-dihydroacridine, 5,10-dihydrophenazine, acridone, phenazaborine, phenophosphazine, phenoselenazine, phenazasiline, azaanthracene, diazaanthracene, azaphenanthrene, diazaphenanthrene and the like; tetracyclic heterocyclic compounds such as hexaazatriphenylene, benzocarbazole, azabenzocarbazole, diazabenzocarbazole, benzonaphthofurane, benzonaphthothiophene and the like; pentacyclic heterocyclic compounds such as dibenzocarbazole, indolocarbazole, indenocarbazole, azaindolocarbazole, diazaindolocarbazole, azaindenocarbazole, diazaindenocarbazole and the like; hexacyclic heterocyclic compounds such as carbazolocarbazole, benzoindolocarbazole, benzoindenocarbazole and the like; and, heptacyclic heterocyclic compounds such as dibenzoindolocarbazole and the like) one or more hydrogen atoms bonding directly to atoms constituting the ring. The hetero ring group includes groups obtained by bonding a plurality of these groups. The hetero ring group optionally has a substituent.

"The halogen atom" denotes a fluorine atom, a chlorine atom, a bromine atom or an iodine atom.

"The amino group" optionally has a substituent, and substituted amino groups (namely, secondary amino groups or tertiary amino groups, more preferably tertiary amino groups) are preferred. The substituent which the amino group has is preferably an alkyl group, a cycloalkyl group, an aryl group or a monovalent hetero ring group. When a plurality of the substituents which the amino group has are present, they may be the same or different and may be combined together to form a ring together with nitrogen atoms to which they are attached.

The substituted amino group includes, for example, a dialkylamino group, a dicycloalkylamino group and a diarylamino group.

The amino group includes, for example, a dimethylamino group, a diethylamino group, a diphenylamino group, a bis(methylphenyl)amino group and a bis(3,5-di-tert-butylphenyl)amino group.

"The alkenyl group" may be any of linear or branched. The number of carbon atoms of the linear alkenyl group, not including the number of carbon atoms of the substituent, is usually 2 to 30, and preferably 3 to 20. The number of carbon atoms of the branched alkenyl group, not including the number of carbon atoms of the substituent, is usually 3 to 30, and preferably 4 to 20.

The number of carbon atoms of "the cycloalkenyl group", not including the number of carbon atoms of the substituent, is usually 3 to 30, and preferably 4 to 20.

The alkenyl group and the cycloalkenyl group optionally have a substituent. The alkenyl group includes, for example, a vinyl group, a 1-propenyl group, a 2-butenyl group, a 3-butenyl group, a 3-pentenyl group, a 4-pentenyl group, a 1-hexenyl group, a 5-hexenyl group and a 7-octenyl group, and groups obtained by substituting a part or all of hydrogen atoms in these groups with a substituent. The cycloalkenyl group includes, for example, a cyclohexenyl group, a cyclohexadienyl group, a cyclooctatrienyl group and a norbornylenyl group, and groups obtained by substituting a part or all of hydrogen atoms in these groups with a substituent.

"The alkynyl group" may be any of linear or branched. The number of carbon atoms of the alkynyl group, not including carbon atoms of the substituent, is usually 2 to 20, and preferably 3 to 20. The number of carbon atoms of the branched alkynyl group, not including carbon atoms of the substituent, is usually 4 to 30, and preferably 4 to 20.

The number of carbon atoms of "the cycloalkynyl group", not including carbon atoms of the substituent, is usually 4 to 30, and preferably 4 to 20.

The alkynyl group and the cycloalkynyl group optionally have a substituent. The alkynyl group includes, for example, an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and groups obtained by substituting a part or all of hydrogen atoms in these groups with a substituent. The cycloalkynyl group includes, for example, a cyclooctynyl group.

"The cross-linkable group" refers to a group capable of generating a new bond by being subjected to a heating treatment, an ultraviolet irradiation treatment, a near-ultraviolet irradiation treatment, a visible light irradiation treatment, an infrared irradiation treatment, a radical reaction and the like. As the cross-linkable group, cross-linkable groups selected from Group A of cross-linkable group (namely, groups represented by any of the formula (XL-1) to the formula (XL-19)) are preferred.

### (Group A of cross-linkable group)

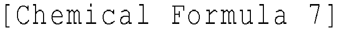

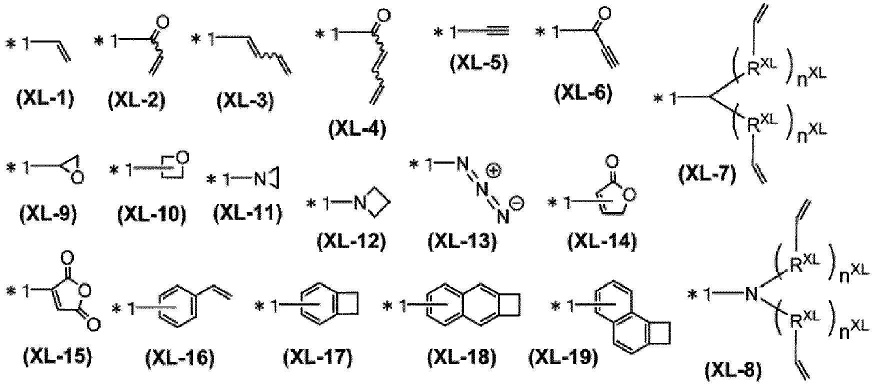

[wherein, R^{XL} represents a methylene group, an oxygen atom or a sulfur atom, and n^{XL} represents an integer of 0 to 5. When a plurality of R^{XL} are present, they may be the same or different. A plurality of n^{XL} may be the same or different. *1 represents a binding position. These cross-linkable groups optionally have a substituent. When a plurality of the substituents are present, they may be the same or different and may be combined together to form a ring together with carbon atoms to which they are attached.]

"The substituent" includes, for example, a halogen atom, a cyano group, an alkyl group, a cycloalkyl group, an aryl group, a monovalent hetero ring group, an alkoxy group, a cycloalkoxy group, an aryloxy group, an amino group, a substituted amino group, an alkenyl group, a cycloalkenyl group, an alkynyl group and a cycloalkynyl group. The substituent may be a cross-linkable group and an electron-attracting group. When a plurality of the substituents are present, they may be combined together to form a ring together with atoms to which they are attached, but it is preferable that they do not form a ring.

In the present specification, calculation of the value of the absolute value of a difference between the energy level of the lowest triplet excited state and the energy level of the lowest singlet excited state (hereinafter, referred to also as "ΔE_{ST}") is carried out by the following method. First, the ground state of a compound is structurally optimized by density-functional approach of B3LYP level. In this procedure, 6-31G* is used as the basis function. Using the resultant structurally optimized structure, ΔE_{ST} of the compound is calculated by B3LYP level time-dependent density-functional approach. In the case of containing an atom to which 6-31G* cannot be applied, LANL2DZ is used for the atom. Calculation is performed using Gaussian09, as the quantum chemistry calculation program.

### <Composition for light emitting device>

The composition for light emitting device of the present embodiment contains a thermally activated delayed fluorescent compound (A) (referred to also simply as "compound (A)") and a compound (B).

The composition for light emitting device of the present embodiment may contain the compound (A) and the compound (B) each singly or in combination of two or more.

In the composition for light emitting device of the present embodiment, the compound (A) and the compound (B) preferably interact physically, chemically or electrically. By this interaction, it becomes possible to improve or adjust, for example, the light emitting property, the charge transporting property or the charge injection property of the composition for light emitting device of the present embodiment, and the light emitting device of the present embodiment is more excellent in light emission efficiency.

In the composition for light emitting device of the present embodiment, if a light emitting material is described as an example, the compound (A) and the compound (B) interact electrically to transfer electrical energy efficiently from the compound (B) to the compound (A), accordingly, the compound (A) can be allowed to emit light more efficiently, and the light emitting device of the present embodiment is more excellent in light emission efficiency.

In the composition for light emitting device of the present embodiment, the content of the compound (A) is usually 0.01 to 99 parts by mass, when the sum of the compound (B) and the compound (A) is taken as 100 parts by mass, and it is preferably 0.1 to 90 parts by mass, more preferably 1 to 90 parts by mass, further preferably 5 to 90 parts by mass, particularly preferably 10 to 85 parts by mass, especially preferably 20 to 80 parts by mass, and, may be 1 to 70 parts by mass, may be 5 to 50 parts by mass and may be 10 to 30 parts by mass, since the light emitting device of the present embodiment is more excellent in light emission efficiency.

In the composition for light emitting device of the present embodiment, it is preferable that the absolute value (ΔE_{ST}(B)) of a difference between the energy level of the lowest triplet excited state and the energy level of the lowest singlet excited state of the compound (B) is larger than the absolute value (ΔE_{ST}(A)) of a difference between the energy level of the lowest triplet excited state and the energy level of the lowest singlet excited state of the compound (A). By this, the compound (B) and the compound (A) easily interact physically, chemically or electrically more efficiently, and the light emitting device of the present embodiment is more excellent in light emission efficiency.

From the above-described standpoint, it is preferable that the lowest excited singlet state (S₁(B)) of the compound (B) is at the energy level higher than that of the lowest excited singlet state (S₁(A)) of the compound (A), since the light emitting device of the present embodiment is more excellent in light emission efficiency. Meanwhile, it is preferable that the lowest excited triplet state (T₁(B)) of the compound (B) is at the energy level higher than that of the lowest excited triplet state (T₁(A)) of the compound (A), since the light emitting device of the present embodiment is more excellent in light emission efficiency.

In the composition for light emitting device of the present embodiment, the absolute value (hereinafter, referred to also as "|EB-AA|") of a difference between EB (the energy value of the maximum peak of the emission spectrum at 25°C of the compound (B)) and AA (the energy value of a peak at the lowest energy side of the absorption spectrum at 25°C of the compound (A)) is preferably 0.60 eV or less, and may be 0.50 eV or less, may be 0.40 eV or less, may be 0.30 eV or less, may be 0.20 eV or less and may be 0.10 eV or less. Meanwhile, |EB-AA| may be 0 eV or more, may be 0.001 eV or more, may be 0.005 eV or more, may be 0.01 eV or more, may be 0.10 eV or more, may be 0.20 eV or more and may be 0.30 eV or more, since the compound (A) and the compound (B) efficiently interact physically, chemically or electrically and the light emitting device of the present embodiment is more excellent in light emission efficiency.

EB is preferably 1.8 eV or more, and may be 2.0 eV or more, may be 2.2 eV or more, may be 2.4 eV or more and may be 2.6 eV or more. EB is preferably 4.5 eV or less, more preferably 4.0 eV or less, further preferably 3.5 eV or less, and may be 3.3 eV or less, may be 3.1 eV or less and may be 3.0 eV or less.

AA is preferably 1.8 eV or more, and may be 2.0 eV or more, may be 2.2 eV or more, may be 2.4 eV or more and may be 2.6 eV or more. AA is preferably 4.5 eV or less, more preferably 4.0 eV or less, further preferably 3.5 eV or less, and may be 3.3 eV or less, may be 3.1 eV or less, may be 3.0 eV or less and may be 2.8 eV or less.

The light emitting device of the present embodiment preferably satisfies AA>EB, since the light emission efficiency is more excellent.

Under the above-mentioned conditions, the relationship between |EB-AA| and the light emitting properties (particularly, light emission efficiency) of the light emitting device is estimated as follows.

The present inventors investigated designs by which the compound (A) and the compound (B) efficiently interact physically, chemically or electrically (particularly, a design for efficient electrical interaction). The present inventors first focused on the fact that the compound (B) is a compound having the small half-value width of the emission spectrum and when the half-value width of the emission spectrum of the compound (B) is small, the overlap between the emission spectrum of the compound (B) and the absorption spectrum of the compound (A) tends to be small. The present inventors considered that an electrical interaction can be obtained more efficiently by increasing the overlap between the emission spectrum of the compound (B) and the absorption spectrum of the compound (A), and focused on |EB-AA|. More specifically, it is estimated that by regulating |EB-AA| to 0.60 eV or less, the overlap between the emission spectrum of the compound (B) and the absorption spectrum of the compound (A) becomes large and the electrical energy of the compound (B) transfers quickly to the compound (A), accordingly, the compound (A) can be allowed to emit light more efficiently, and resultantly, the light emitting device are excellent in light emitting properties (particularly, light emission efficiency).

The energy value of the maximum peak of the emission spectrum and the energy value of a peak at the lowest energy side of the absorption spectrum of the compound can be determined by measuring the wavelength of the maximum peak of the emission spectrum and the wavelength of a peak at the lowest energy side of the absorption spectrum of the compound, then, converting the resultant peak wavelengths into the energy value.

The wavelength of the maximum peak of the emission spectrum at room temperature of the compound can be evaluated by dissolving the compound in an organic solvent such as xylene, toluene, chloroform, tetrahydrofuran and the like to prepare a dilute solution (1×10⁻⁶% by mass to 1×10⁻³% by mass), and measuring the PL spectrum of the dilute solution at room temperature. The organic solvent for dissolving the compound is preferably xylene.

The wavelength of a peak at the lowest energy side of the absorption spectrum at room temperature of the compound can be evaluated by dissolving the compound in an organic solvent such as xylene, toluene, chloroform, tetrahydrofuran and the like to prepare a dilute solution (1×10⁻⁶% by mass to 1×10⁻³% by mass), and measuring the ultraviolet visible absorption spectrum of the dilute solution at room temperature. The organic solvent for dissolving the compound is preferably xylene.

### [Compound (B)]

The compound (B) is a compound having a condensed hetero ring skeleton (b) containing a boron atom and at least one selected from the group consisting of an oxygen atom, a sulfur atom, a selenium atom, an sp³ carbon atom and a nitrogen atom in the ring.

In the compound (B), if the condensed hetero ring skeleton (b) contain nitrogen atoms, it is preferable that at least one of nitrogen atoms contained in the condensed hetero ring skeleton (b) is a nitrogen atom not forming a double bond, and it is more preferable that all nitrogen atoms contained in the condensed hetero ring skeleton (b) are nitrogen atoms not forming a double bond.

The number of carbon atoms of the condensed hetero ring skeleton (b), not including the number of carbon atoms of the substituent, is usually 1 to 60, preferably 5 to 40, and more preferably 10 to 25.

The number of hetero atoms of the condensed hetero ring skeleton (b), not including the number of hetero atoms of the substituent, is usually 2 to 30, preferably 2 to 15, more preferably 2 to 10, further preferably 2 to 5, and particularly preferably 2 or 3.

The number of boron atoms of the condensed hetero ring skeleton (b), not including the number of boron atoms of the substituent, is usually 1 to 10, preferably 1 to 5, more preferably 1 to 3, and further preferably 1.

The total number of an oxygen atom, a sulfur atom, a selenium atom, an sp³ carbon atom and a nitrogen atom of the condensed hetero ring skeleton (b), not including the number of the atoms of the substituent, is usually 1 to 20, preferably 1 to 10, more preferably 1 to 5, further preferably 1 to 3, and particularly preferably 2.

The condensed hetero ring skeleton (b) preferably contains a boron atom and at least one selected from the group consisting of an oxygen atom, a sulfur atom and a nitrogen atom in the ring, more preferably contains a boron atom and a nitrogen atom in the ring, and further preferably contains a boron atom and a nitrogen atom not forming a double bond in the ring, since the light emitting device of the present embodiment is more excellent in light emission efficiency.

The condensed hetero ring skeleton (b) is preferably a tri to dodecacyclic condensed hetero ring skeleton, more preferably a tri to hexacyclic condensed hetero ring skeleton, and further preferably a pentacyclic condensed hetero ring skeleton, since the light emitting device of the present embodiment is more excellent in light emission efficiency.

The condensed hetero ring skeleton (b) can also be referred to as a compound having a hetero ring group (b') containing a condensed hetero ring skeleton (b).

The hetero ring group (b') may be a group obtained by removing from a polycyclic heterocyclic compound containing a boron atom and at least one selected from the group consisting of an oxygen atom, a sulfur atom, a selenium atom, an sp³ carbon atom and a nitrogen atom in the ring one or more hydrogen atoms bonding directly to atoms constituting the ring, and the group optionally has a substituent. In the hetero ring group (b'), the polycyclic heterocyclic compound is preferably a polycyclic heterocyclic compound containing a boron atom and at least one selected from the group consisting of an oxygen atom, a sulfur atom and a nitrogen atom in the ring, more preferably a polycyclic heterocyclic compound containing a boron atom and a nitrogen atom in the ring, and further preferably a polycyclic heterocyclic compound containing a boron atom and a nitrogen atom not forming a double bond in the ring. In the hetero ring group (b'), the polycyclic heterocyclic compound is preferably a tri to dodecacyclic heterocyclic compound, more preferably a tri to hexacyclic heterocyclic compound, and further preferably a pentacyclic heterocyclic compound.

The substituent which a hetero ring group (b') optionally has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an alkoxy group, a cycloalkoxy group, an aryl group, a monovalent hetero ring group or a substituted amino group, and further preferably an alkyl group, an aryl group or a substituted amino group, and these groups optionally further have a substituent.

The aryl group as the substituent which a hetero ring group (b') optionally has is preferably a group obtained by removing from a monocyclic or dicyclic to hexacyclic aromatic hydrocarbon one hydrogen atom bonding directly to an atom constituting the ring, more preferably a group obtained by removing from a monocyclic, dicyclic or tricyclic aromatic hydrocarbon one hydrogen atom bonding directly to an atom constituting the ring, further preferably a group obtained by removing from benzene, naphthalene, anthracene, phenanthrene or fluorene one hydrogen atom bonding directly to an atom constituting the ring, and particularly preferably a phenyl group, and these groups optionally have a substituent.

The monovalent hetero ring group as the substituent which a hetero ring group (b') optionally has is preferably a group obtained by removing from a monocyclic or dicyclic to hexacyclic heterocyclic compound one hydrogen atom bonding directly to an atom constituting the ring, more preferably a group obtained by removing from a monocyclic, dicyclic or tricyclic heterocyclic compound one hydrogen atom bonding directly to an atom constituting the ring, further preferably a group obtained by removing from pyridine, diazabenzene, triazine, azanaphthalene, diazanaphthalene, carbazole, dibenzofuran, dibenzothiophene, phenoxazine or phenothiazine one hydrogen atom bonding directly to an atom constituting the ring, and particularly preferably a group obtained by removing from pyridine, diazabenzene or triazine one hydrogen atom bonding directly to an atom constituting the ring, and these groups optionally have a substituent.

In the substituted amino group as the substituent which a hetero ring group (b') optionally has, the substituent which the amino group has is preferably an aryl group or a monovalent hetero ring group, and more preferably an aryl group, and these groups optionally further have a substituent. The examples and preferable ranges of the aryl group and the monovalent hetero ring group as the substituent which the amino group has are the same as the examples and preferable ranges of the aryl group and the monovalent hetero ring group as the substituent which a hetero ring group (b') optionally has, respectively.

The substituent which the substituent which a hetero ring group (b') optionally has optionally further has is preferably a halogen atom, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, and further preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which a hetero ring group (b') optionally has optionally further has are the same as the examples and preferable ranges of the aryl group, the monovalent hetero ring group and the substituted amino group as the substituent which a hetero ring group (b') optionally has, respectively.

"The nitrogen atom not forming a double bond" means a nitrogen atom that is single-bonded to each of the other three atoms.

The phrase "containing a nitrogen atom not forming a double bond in the ring" means that a group represented by -N(-R^{N})- (wherein, R^{N} represents a hydrogen atom or a substituent) or the formula: 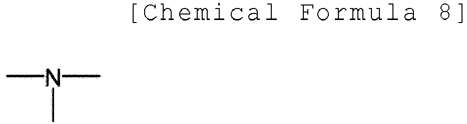 is contained in the ring.

The compound (B) is preferably a thermally activated delayed fluorescent (TADF) compound, since the light emitting device of the present embodiment is more excellent in light emission efficiency.

ΔE_{ST} of the compound (B) is preferably 0.50 eV or less, since the light emitting device of the present embodiment is more excellent in light emission efficiency. Meanwhile, ΔE_{ST} of the compound (B) may be 0.001 eV or more, may be 0.01 eV or more, may be 0.10 eV or more, may be 0.20 eV or more, may be 0.30 eV or more and may be 0.40 eV or more.

The compound (B) is preferably a low molecular weight compound.

The molecular weight of the compound (B) is preferably 1×10² to 5×10³, more preferably 2×10² to 3×10³, further preferably 3×10² to 1.5×10³, and particularly preferably 4×10² to 1×10³.

The compound (B) is preferably a compound represented by the formula (1-1), the formula (1-2) or the formula (1-3), more preferably a compound represented by the formula (1-2) or the formula (1-3), and further preferably a compound represented by the formula (1-2), since the light emitting device of the present embodiment is more excellent in light emission efficiency.

Ar¹, Ar² and Ar³ are each a group obtained by removing from a monocyclic, dicyclic or tricyclic aromatic hydrocarbon or a monocyclic, dicyclic or tricyclic heterocyclic compound one or more hydrogen atoms bonding directly to atoms constituting the ring, more preferably a group obtained by removing from a monocyclic aromatic hydrocarbon or a monocyclic heterocyclic compound one or more hydrogen atoms bonding directly to atoms constituting the ring, further preferably a group obtained by removing from benzene, pyridine or diazabenzene one or more hydrogen atoms bonding directly to atoms constituting the ring, and particularly preferably a group obtained by removing from benzene one or more hydrogen atoms bonding directly to atoms constituting the ring, since the light emitting device of the present embodiment is more excellent in light emission efficiency, and these groups optionally have a substituent.

The examples and preferable ranges of the substituent which Ar¹, Ar² and Ar³ optionally have are the same as the examples and preferable ranges of the substituent which a hetero ring group (b') optionally has.

Y¹ is preferably an oxygen atom, a sulfur atom, a group represented by -N(Ry)- or a methylene group, more preferably an oxygen atom, a sulfur atom or a group represented by -N(Ry)-, and further preferably a group represented by -N(Ry)-, and these groups optionally have a substituent.

Y² and Y³ are each preferably a single bond, an oxygen atom, a sulfur atom, a group represented by -N(Ry)- or a methylene group, more preferably a single bond, an oxygen atom, a sulfur atom or a group represented by -N(Ry)-, further preferably an oxygen atom, a sulfur atom or a group represented by -N(Ry)-, and particularly preferably a group represented by -N(Ry)-, and these groups optionally have a substituent.

It is preferable that all of Y¹, Y² and Y³ represent an oxygen atom, a sulfur atom or a group represented by -N(Ry)-, and it is more preferable that all of Y¹, Y² and Y³ represent a group represented by -N(Ry)-, since the light emitting device of the present embodiment is more excellent in light emission efficiency.

The examples and preferable ranges of the substituent which Y¹, Y² and Y³ optionally have are the same as the examples and preferable ranges of the substituent which a hetero ring group (b') optionally has.

Ry is preferably an alkyl group, a cycloalkyl group, an aryl group or a monovalent hetero ring group, more preferably an aryl group or a monovalent hetero ring group, and further preferably an aryl group, and these groups optionally have a substituent.

The examples and preferable ranges of the aryl group and the monovalent hetero ring group represented by Ry are the same as the examples and preferable ranges of the aryl group and the monovalent hetero ring group as the substituent which a hetero ring group (b') optionally has, respectively.

The examples and preferable ranges of the substituent which Ry optionally has are the same as the examples and preferable ranges of the substituent which a hetero ring group (b') optionally has.

Ry may be bonded directly or via a connecting group to Ar¹, Ar² or Ar³, but it is preferable that it is not bonded. The connecting group includes, for example, a group represented by -O-, a group represented by -S-, a group represented by -N(Ry)-, an alkylene group, a cycloalkylene group, an arylene group and a divalent hetero ring group, and is preferably a group represented by -O-, a group represented by -S-, a group represented by -N(Ry)- or a methylene group, and these groups optionally have a substituent.

As the compound (B), compounds represented by the following formulae and compounds B1 to B3 described later are exemplified.

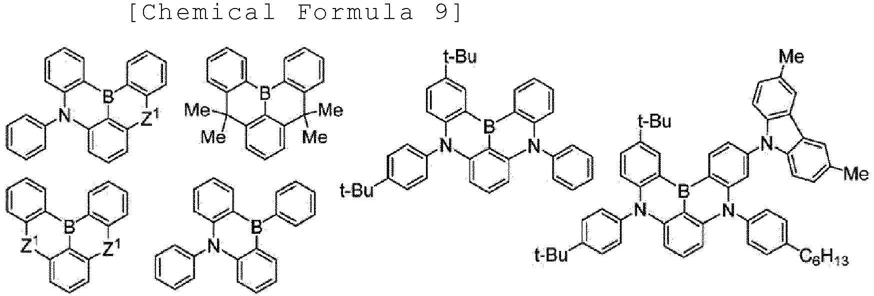

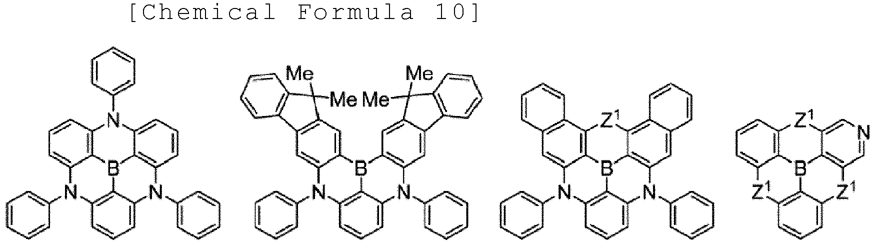

In the formulae, Z¹ represents an oxygen atom or a sulfur atom.

### [Compound (A)]

The compound (A) is a compound having a thermally activated delayed fluorescent (TADF) property.

The compound (A) is a compound different from the compound (B), and may be, for example, a compound having no condensed hetero ring skeleton (b).

ΔE_{ST} of the compound (A) is usually 0.50 eV or less. ΔE_{ST} of the compound (A) is preferably 0.45 eV or less, more preferably 0.40 eV or less, further preferably 0.35 eV or less, particularly preferably 0.30 eV or less, especially preferably 0.25 eV or less, and especially more preferably 0.20 eV or less, since the light emitting device of the present embodiment is more excellent in light emission efficiency. Meanwhile, ΔE_{ST} of the compound (A) may be 0.001 eV or more, may be 0.005 eV or more, may be 0.01 eV or more and may be 0.05 eV or more.

The energy value (EA) of the maximum peak of the emission spectrum at room temperature of the compound (A) is preferably 1.6 eV or more, and may be 1.8 eV or more, may be 2.0 eV or more, may be 2.1 eV or more, may be 2.2 eV or more, and may be 2.3 eV or more. Meanwhile, EA is preferably 4.5 eV or less, more preferably 4.0 eV or less, further preferably 3.5 eV or less, particularly preferably 3.3 eV or less, and may be 3.1 eV or less, preferably may be 3.0 eV or less, may be 2.9 eV or less, may be 2.8 eV or less, may be 2.7 eV or less, may be 2.6 eV or less and may be 2.5 eV or less.

The compound (A) is preferably a low molecular weight compound.

The molecular weight of the compound (A) is preferably 1×10² to 1×10⁴, more preferably 2×10² to 5×10³, further preferably 3×10² to 3×10³, and particularly preferably 4×10² to 1.5×10³.

### [Compound represented by the formula (T-1)]

The compound (A) is preferably a compound represented by the formula (T-1), since the light emitting device of the present embodiment is more excellent in light emission efficiency.

n^{T1} is usually an integer of 0 or more and 10 or less, and it is preferably an integer of 0 or more and 5 or less, more preferably an integer of 0 or more and 3 or less, further preferably an integer of 0 or more and 2 or less, and particularly preferably 0 or 1, since the light emitting device of the present embodiment is more excellent in light emission efficiency.

n^{T2} is usually an integer of 1 or more and 10 or less, and it is preferably an integer of 1 or more and 7 or less, more preferably an integer of 1 or more and 5 or less, further preferably an integer of 1 or more and 4 or less, and may be 2 or 3, since the light emitting device of the present embodiment is excellent in light emission efficiency.

In the monovalent hetero ring group containing a nitrogen atom not forming a double bond in the ring and not containing a group represented by =N-, a group represented by -C(=O)-, a group represented by -S(=O)- and a group represented by -S(=O)₂- in the ring (hereinafter, referred to as "monovalent donor type hetero ring group"), the number of the nitrogen atom not forming a double bond constituting the ring is usually 1 to 10, preferably 1 to 5, more preferably 1 to 3, and further preferably 1 or 2.

In the monovalent donor type hetero ring group, the number of carbon atoms constituting the ring is usually 1 to 60, preferably 3 to 50, more preferably 5 to 40, further preferably 7 to 30, and particularly preferably 10 to 25.

In the monovalent donor type hetero ring group, the number of hetero atoms constituting the ring is usually 1 to 30, preferably 1 to 10, more preferably 1 to 5, and further preferably 1 to 3.

The monovalent donor type hetero ring group is preferably a group obtained by removing from a heterocyclic compound containing no condensed hetero ring skeleton (b) one hydrogen atom bonding directly to an atom constituting the ring, and this group optionally has a substituent. In the monovalent donor type hetero ring group, the heterocyclic compound containing no condensed hetero ring skeleton (b) includes, for example, heterocyclic compounds not containing a boron atom and a nitrogen atom in the ring, among heterocyclic compounds described in the section of the hetero ring group described above.

The monovalent donor type hetero ring group includes, for example, monovalent hetero ring groups containing a nitrogen atom not forming a double bond in the ring and not containing a group represented by =N-, a group represented by -C(=O)-, a group represented by -S(=O)- and a group represented by -S(=O)₂- in the ring, among hetero ring groups described in the section of the hetero ring group described above. The monovalent donor type hetero ring group is preferably a group obtained by removing from a polycyclic heterocyclic compound containing a nitrogen atom not forming a double bond in the ring and not containing a group represented by =N-, a group represented by -C(=O)-, a group represented by -S(=O)- and a group represented by -S(=O)₂- in the ring (preferably, a polycyclic heterocyclic compound containing no condensed hetero ring skeleton (b)) one hydrogen atom bonding directly to an atom (preferably a carbon atom or a nitrogen atom, more preferably a nitrogen atom) constituting the ring, more preferably a group obtained by removing from a tricyclic to pentacyclic heterocyclic compound containing a nitrogen atom not forming a double bond in the ring and not containing a group represented by =N-, a group represented by -C(=O)-, a group represented by -S(=O)- and a group represented by -S(=O)₂- in the ring (preferably, a tricyclic to pentacyclic heterocyclic compound containing no condensed hetero ring skeleton (b)) one hydrogen atom bonding directly to an atom (preferably a carbon atom or a nitrogen atom, more preferably a nitrogen atom) constituting the ring, further preferably a group obtained by removing from carbazole, phenoxazine, phenothiazine, 9,10-dihydroacridine, 5,10-dihydrophenazine, benzocarbazole, dibenzocarbazole, indolocarbazole or indenocarbazole one hydrogen atom bonding directly to an atom (preferably a carbon atom or a nitrogen atom, more preferably a nitrogen atom) constituting the ring, and particularly preferably a group obtained by removing from carbazole, indolocarbazole or indenocarbazole one hydrogen atom bonding directly to an atom (preferably a carbon atom or a nitrogen atom, more preferably a nitrogen atom) constituting the ring, since the light emitting device of the present embodiment is more excellent in light emission efficiency, and these groups optionally have a substituent.

The examples and preferable ranges of the substituted amino group represented by Ar^{T1} are the same as the examples and preferable ranges of the substituted amino group as the substituent which Ar^{T1} optionally has described later.

The substituent which Ar^{T1} optionally has is preferably an alkyl group, a cycloalkyl group, an alkoxy group, a cycloalkoxy group, an aryl group, an aryloxy group, a monovalent hetero ring group, a substituted amino group, a halogen atom or a cyano group, more preferably an alkyl group, a cycloalkyl group, an alkoxy group, a cycloalkoxy group, an aryl group, a monovalent hetero ring group or a substituted amino group, and further preferably an alkyl group, a cycloalkyl group, an aryl group, a monovalent hetero ring group or a substituted amino group, and these groups optionally further have a substituent.

The aryl group as the substituent which Ar^{T1} optionally has is preferably a group obtained by removing from a monocyclic or dicyclic to heptacyclic aromatic hydrocarbon one hydrogen atom bonding directly to an atom constituting the ring, more preferably a group obtained by removing from a monocyclic or dicyclic to pentacyclic (preferably, a monocyclic, dicyclic or tricyclic) aromatic hydrocarbon one hydrogen atom bonding directly to an atom constituting the ring, further preferably a group obtained by removing from benzene, naphthalene, anthracene, phenanthrene or fluorene one hydrogen atom bonding directly to an atom constituting the ring, and particularly preferably a phenyl group, and these groups optionally have a substituent.

The monovalent hetero ring group as the substituent which Ar^{T1} optionally has is preferably a group obtained by removing from a heterocyclic compound containing no condensed hetero ring skeleton (b) one hydrogen atom bonding directly to an atom constituting the ring, and this group optionally has a substituent. In the monovalent hetero ring group as the substituent which Ar^{T1} optionally has, the heterocyclic compound containing no condensed hetero ring skeleton (b) includes heterocyclic compounds not containing a boron atom and a nitrogen atom in the ring, among heterocyclic compounds described in the section of the hetero ring group described above. The monovalent hetero ring group as the substituent which Ar^{T1} optionally has is preferably a group obtained by removing from a monocyclic or dicyclic to heptacyclic heterocyclic compound (preferably, a monocyclic or di to heptacyclic heterocyclic compound containing no condensed hetero ring skeleton (b)) one hydrogen atom bonding directly to an atom constituting the ring, more preferably a group obtained by removing from a monocyclic or dicyclic to pentacyclic (preferably monocyclic, dicyclic or tricyclic) heterocyclic compound (preferably a monocyclic or dicyclic to pentacyclic heterocyclic compound containing no condensed hetero ring skeleton (b), more preferably a monocyclic, dicyclic or tricyclic heterocyclic compound containing no condensed hetero ring skeleton (b)) one or more hydrogen atoms bonding directly to atoms constituting the ring, further preferably a group obtained by removing from pyridine, diazabenzene, triazine, azanaphthalene, diazanaphthalene, dibenzofuran, dibenzothiophene, carbazole, phenoxazine, phenothiazine, 9,10-dihydroacridine, 5,10-dihydrophenazine, benzocarbazole, dibenzocarbazole, indolocarbazole or indenocarbazole one or more hydrogen atoms bonding directly to atoms constituting the ring, and particularly preferably a group obtained by removing from pyridine, diazabenzene, triazine, dibenzofuran, dibenzothiophene or carbazole one or more hydrogen atoms bonding directly to atoms constituting the ring, and these groups optionally further have a substituent.

In the substituted amino group as the substituent which Ar^{T1} optionally has, the substituent which the amino group has is preferably an aryl group or a monovalent hetero ring group, and more preferably an aryl group, and these groups optionally further have a substituent. The examples and preferable ranges of the aryl group as the substituent which the amino group has are the same as the examples and preferable ranges of the aryl group as the substituent which Ar^{T1} optionally has. The examples and preferable ranges of the monovalent hetero ring group as the substituent which the amino group has are the same as the examples and preferable ranges of the monovalent hetero ring group as the substituent which Ar^{T1} optionally has.

The substituent which the substituent which Ar^{T1} optionally has optionally further has is preferably an alkyl group, a cycloalkyl group, an alkoxy group, a cycloalkoxy group, an aryl group, an aryloxy group, a monovalent hetero ring group, a substituted amino group, a halogen atom or a cyano group, more preferably an alkyl group, a cycloalkyl group, an aryl group, a monovalent hetero ring group or a substituted amino group, and further preferably an alkyl group or an aryl group, and these groups optionally further have a substituent, but it is preferable that they do not further have a substituent.

The examples and preferable ranges of the aryl group, the monovalent hetero ring group and the substituted amino group as the substituent which the substituent which Ar^{T1} optionally has optionally further has are the same as the examples and preferable ranges of the aryl group, the monovalent hetero ring group and the substituted amino group as the substituent which Ar^{T1} optionally has, respectively.

It is preferable that at least one of Ar^{T1} is a monovalent donor type hetero ring group optionally having a substituent, since the light emitting device of the present embodiment is more excellent in light emission efficiency.

Ar^{T1} is preferably a monovalent donor type hetero ring group optionally having a substituent, since the light emitting device of the present embodiment is more excellent in light emission efficiency.

L^{T1} is preferably an alkylene group, a cycloalkylene group, an arylene group or a divalent hetero ring group, more preferably an arylene group or a divalent hetero ring group, and further preferably an arylene group, since the light emitting device of the present embodiment is more excellent in light emission efficiency, and these groups optionally have a substituent.

The arylene group represented by L^{T1} is preferably a group obtained by removing from a monocyclic or dicyclic to hexacyclic aromatic hydrocarbon two hydrogen atoms bonding directly to atoms constituting the ring, more preferably a group obtained by removing from a monocyclic, dicyclic or tricyclic aromatic hydrocarbon two hydrogen atoms bonding directly to atoms constituting the ring, further preferably a group obtained by removing from benzene, naphthalene, anthracene, phenanthrene or fluorene two hydrogen atoms bonding directly to atoms constituting the ring, and particularly preferably a phenylene group, and these groups optionally have a substituent.

The divalent hetero ring group represented by L^{T1} is preferably a group obtained by removing from a heterocyclic compound containing no condensed hetero ring skeleton (b) two hydrogen atoms bonding directly to atoms constituting the ring. In the divalent hetero ring group represented by L^{T1}, the heterocyclic compound containing no condensed hetero ring skeleton (b) includes heterocyclic compounds not containing a boron atom and a nitrogen atom in the ring, among heterocyclic compounds described in the section of the hetero ring group described above. The divalent hetero ring group represented by L^{T1} is preferably a group obtained by removing from a monocyclic or dicyclic to hexacyclic heterocyclic compound (preferably, a monocyclic or dicyclic to hexacyclic heterocyclic compound containing no condensed hetero ring skeleton (b)) two hydrogen atoms bonding directly to atoms (preferably, carbon atoms) constituting the ring, more preferably a group obtained by removing from a monocyclic, dicyclic or tricyclic heterocyclic compound (preferably, a monocyclic, dicyclic or tricyclic heterocyclic compound containing no condensed hetero ring skeleton (b)) two hydrogen atoms bonding directly to atoms (preferably, carbon atoms) constituting the ring, further preferably a group obtained by removing from pyridine, diazabenzene, triazine, azanaphthalene, diazanaphthalene, carbazole, dibenzofuran, dibenzothiophene, phenoxazine or phenothiazine two hydrogen atoms bonding directly to atoms (preferably, carbon atoms) constituting the ring, and particularly preferably a group obtained by removing from pyridine, diazabenzene or triazine two hydrogen atoms bonding directly to atoms (preferably, carbon atoms) constituting the ring, and these groups optionally have a substituent.

The examples and preferable ranges of the substituent which L^{T1} optionally has are the same as the examples and preferable ranges of the substituent which Ar^{T1} optionally has.

In Ar^{T2}, the aromatic hydrocarbon group having an electron-attracting group means an aromatic hydrocarbon group having an electron-attracting group as the substituent, and this group optionally has a substituent other than the electron-attracting group.

In the aromatic hydrocarbon group containing an electron-attracting group, the number of the electron-attracting group which the aromatic hydrocarbon group has is usually 1 to 20, preferably 1 to 10, more preferably 1 to 7, further preferably 1 to 5, and particularly preferably 1 to 3.

The electron-attracting group includes, for example, an alkyl group having a fluorine atom as the substituent, a fluorine atom, a cyano group, a nitro group, an acyl group and a carboxyl group, and is preferably a cyano group, an alkyl group having a fluorine atom as the substituent or a fluorine atom, and more preferably a cyano group.

The alkyl group having a fluorine atom as the substituent is preferably a trifluoromethyl group, a pentafluoroethyl group, a perfluorobutyl group, a perfluorohexyl group or a perfluorooctyl group.

The aromatic hydrocarbon group in the aromatic hydrocarbon group containing an electron-attracting group is preferably a group obtained by removing from a monocyclic or dicyclic to hexacyclic aromatic hydrocarbon one or more hydrogen atoms bonding directly to atoms constituting the ring, more preferably a group obtained by removing from a monocyclic, dicyclic or tricyclic aromatic hydrocarbon one or more hydrogen atoms bonding directly to atoms constituting the ring, further preferably a group obtained by removing from benzene, naphthalene, anthracene, phenanthrene or fluorene one or more hydrogen atoms bonding directly to atoms constituting the ring, and particularly preferably a group obtained by removing from benzene one or more hydrogen atoms bonding directly to atoms constituting the ring, and these groups optionally have a substituent.

In the aromatic hydrocarbon group containing a group represented by -C(=O)- in the ring represented by Ar^{T2}, the number of the group represented by -C(=O)- constituting the ring is usually 1 to 10, preferably 1 to 7, more preferably 1 to 5, and further preferably 1 to 3.

The aromatic hydrocarbon group containing a group represented by -C(=O)- in the ring represented by Ar^{T2} includes aromatic hydrocarbon groups containing a group represented by -C(=O)- in the ring among aromatic hydrocarbon groups described in the section of the aromatic hydrocarbon group described above, and is preferably a group obtained by removing from a dicyclic or tricyclic aromatic hydrocarbon containing a group represented by -C(=O)- in the ring one or more hydrogen atoms bonding directly to atoms constituting the ring, more preferably a group obtained by removing from naphthoquinone, anthraquinone, phenanthoquinone, indenone, fluorenone or tetralone one or more hydrogen atoms bonding directly to atoms constituting the ring, and further preferably a group obtained by removing from anthraquinone, phenanthoquinone or fluorenone one or more hydrogen atoms bonding directly to atoms constituting the ring, and these groups optionally have a substituent.

In the hetero ring group containing at least one group selected from the group consisting of a group represented by =N-, a group represented by -C(=O)-, a group represented by -S(=O)- and a group represented by -S(=O)₂- in the ring represented by Ar^{T2} (hereinafter, referred to also as "acceptor type hetero ring group"), the total number of a group represented by =N-, a group represented by - C(=O)-, a group represented by -S(=O)- and a group represented by -S(=O)₂- constituting the ring is usually 1 to 20, preferably 1 to 10, more preferably 1 to 5, and further preferably 1 to 3.

In the acceptor type hetero ring group, the number of carbon atoms constituting the ring is usually 1 to 60, preferably 2 to 40, and more preferably 3 to 20.

In the acceptor type hetero ring group, the number of hetero atoms constituting the ring is usually 1 to 30, preferably 1 to 10, more preferably 1 to 5, and further preferably 1 to 3.

The acceptor type hetero ring group is preferably a group obtained by removing from a heterocyclic compound containing no condensed hetero ring skeleton (b) one or more hydrogen atoms bonding directly to atoms constituting the ring, and this group optionally has a substituent. In the acceptor type hetero ring group, the heterocyclic compound containing no condensed hetero ring skeleton (b) includes, for example, heterocyclic compounds not containing a boron atom and a nitrogen atom in the ring among heterocyclic compounds described in the section of the hetero ring group described above.

The acceptor type hetero ring group includes, for example, groups obtained by removing from a heterocyclic compound containing at least one group selected from the group consisting of a group represented by =N-, a group represented by -C(=O)-, a group represented by -S(=O)- and a group represented by -S(=O)₂- in the ring (preferably, a heterocyclic compound containing no condensed hetero ring skeleton (b)) among heterocyclic compounds described in the section of the hetero ring group described above one or more hydrogen atoms bonding directly to atoms (preferably, carbon atoms) constituting the ring, and is preferably a group obtained by removing from a monocyclic or di to pentacyclic heterocyclic compound containing at least one group selected from the group consisting of a group represented by =N-, a group represented by -C(=O)-, a group represented by -S(=O)- and a group represented by -S(=O)₂- in the ring (preferably, a monocyclic or di to pentacyclic heterocyclic compound containing no condensed hetero ring skeleton (b)) one or more hydrogen atoms bonding directly to atoms (preferably, carbon atoms) constituting the ring, more preferably a group obtained by removing from a monocyclic, dicyclic or tricyclic heterocyclic compound containing at least one group selected from the group consisting of a group represented by =N-, a group represented by -C(=O)-, a group represented by -S(=O)- and a group represented by -S(=O)₂- in the ring (preferably, a monocyclic, dicyclic or tricyclic heterocyclic compound containing no condensed hetero ring skeleton (b)) one or more hydrogen atoms bonding directly to atoms (preferably, carbon atoms) constituting the ring, further preferably a group obtained by removing from oxadiazole, thiadiazole, pyridine, diazabenzene, triazine, azanaphthalene, diazanaphthalene, dibenzothiophene dioxide, dibenzothiophene oxide, dibenzopyranone, azaanthracene, diazaanthracene, azaphenanthrene, diazaphenanthrene, azacarbazole, diazacarbazole or acridone one or more hydrogen atoms bonding directly to atoms (preferably, carbon atoms) constituting the ring, particularly preferably a group obtained by removing from oxadiazole, thiadiazole, pyridine, diazabenzene, triazine, dibenzothiophene dioxide, dibenzothiophene oxide or dibenzopyranone one or more hydrogen atoms bonding directly to atoms (preferably, carbon atoms) constituting the ring, especially preferably a group obtained by removing from oxadiazole, thiadiazole, pyridine, diazabenzene, triazine or dibenzopyranone one or more hydrogen atoms bonding directly to atoms (preferably, carbon atoms) constituting the ring, and especially more preferably a group obtained by removing from pyridine, diazabenzene or triazine one or more hydrogen atoms bonding directly to atoms constituting the ring, and these groups optionally have a substituent.

The acceptor type hetero ring group is preferably a hetero ring group containing at least one group selected from the group consisting of a group represented by -C(=O)-, a group represented by -S(=O)₂- and a group represented by =N- in the ring, more preferably a hetero ring group containing at least one group selected from the group consisting of a group represented by -C(=O)- and a group represented by =N- in the ring, and further preferably a hetero ring group containing a group represented by =N- in the ring, since the light emitting device of the present embodiment is more excellent in light emission efficiency, and these groups optionally have a substituent.

Ar^{T2} is preferably a group represented by - C(=O)-, a group represented by -S(=O)₂-, an aromatic hydrocarbon group having an electron-attracting group, or an acceptor type hetero ring group, more preferably a group represented by - S(=O)₂-, an aromatic hydrocarbon group having an electron-attracting group, or an acceptor type hetero ring group, further preferably an aromatic hydrocarbon group having an electron-attracting group or acceptor type hetero ring group, and particularly preferably an aromatic hydrocarbon group having an electron-attracting group, since the light emitting device of the present embodiment is more excellent in light emission efficiency, and these groups optionally have a substituent.

The substituent which Ar^{T2} optionally has is preferably an alkyl group, a cycloalkyl group, an alkoxy group, a cycloalkoxy group, an aryl group, an aryloxy group, a monovalent hetero ring group or an electron-attracting group, more preferably an alkyl group, a cycloalkyl group, an alkoxy group, a cycloalkoxy group, an aryl group, a monovalent hetero ring group or an electron-attracting group, further preferably an alkyl group, an aryl group, a monovalent hetero ring group or an electron-attracting group, and particularly preferably an alkyl group, an aryl group or an electron-attracting group, and these groups optionally have a substituent.

The examples and preferable ranges of the aryl group as the substituent which Ar^{T2} optionally has are the same as the examples and preferable ranges of the aryl group as the substituent which Ar^{T1} optionally has.

The monovalent hetero ring group as the substituent which Ar^{T2} optionally has is preferably a monovalent hetero ring group which is a group obtained by removing from a heterocyclic compound containing no condensed hetero ring skeleton (b) one hydrogen atom bonding directly to an atom constituting the ring and other than the monovalent donor type hetero ring group, and this group optionally further has a substituent. In the monovalent hetero ring group as the substituent which Ar^{T2} optionally has, the heterocyclic compound containing no condensed hetero ring skeleton (b) includes heterocyclic compounds not containing a boron atom and a nitrogen atom in the ring among heterocyclic compounds described in the section of the hetero ring group described above. The monovalent hetero ring group as the substituent which Ar^{T2} optionally has includes, for example, a hetero ring group which is a group obtained by removing from a heterocyclic compound containing no condensed hetero ring skeleton (b) one hydrogen atom bonding directly to an atom constituting the ring and other than the monovalent donor type hetero ring group among hetero ring groups described in the section of the hetero ring group described above, and is preferably a group obtained by removing from a monocyclic or dicyclic to hexacyclic heterocyclic compound (preferably, a monocyclic or dicyclic to hexacyclic heterocyclic compound containing no condensed hetero ring skeleton (b)) one hydrogen atom bonding directly to an atom constituting the ring (preferably, a hetero ring group other than the monovalent donor type hetero ring group), more preferably a group obtained by removing from a monocyclic, dicyclic or tricyclic heterocyclic compound (preferably, a monocyclic, dicyclic or tricyclic heterocyclic compound containing no condensed hetero ring skeleton (b)) one or more hydrogen atoms bonding directly to atoms constituting the ring (preferably, a hetero ring group other than the monovalent donor type hetero ring group), further preferably a group obtained by removing from pyridine, diazabenzene, triazine, azanaphthalene, diazanaphthalene, dibenzofuran, dibenzothiophene, azacarbazole or diazacarbazole one or more hydrogen atoms bonding directly to atoms constituting the ring, and particularly preferably a group obtained by removing from pyridine, diazabenzene or triazine one or more hydrogen atoms bonding directly to atoms constituting the ring, and these groups optionally further have a substituent.

The examples and preferable ranges of the substituent which the substituent which Ar^{T2} optionally has optionally further has are the same as the examples and preferable ranges of the substituent which the substituent which Ar^{T1} optionally has optionally further has.

The compound (A) includes, for example, compounds represented by the following formulae, and compounds T5, T6, T8 to T10 and T12 described later. In the formulae, Z¹ represents an oxygen atom or a sulfur atom. Z² represents a group represented by -N= or a group represented by -CH=. Z³ represents a group represented by -C(=O)- or a group represented by -S(=O)₂-. When a plurality of Z¹ to Z³ are present, they may be the same or different at each occurrence.

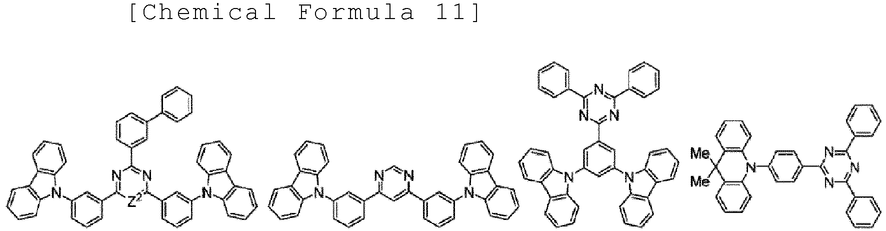

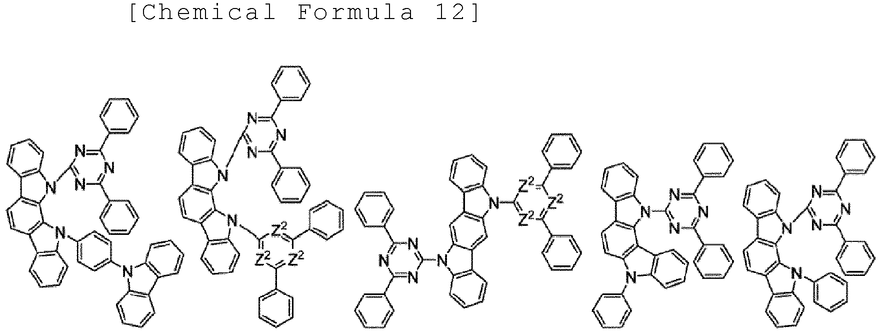

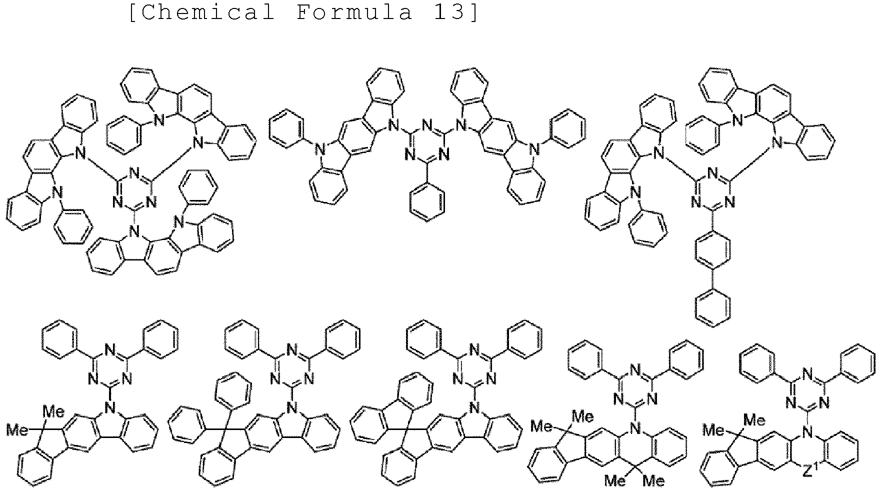

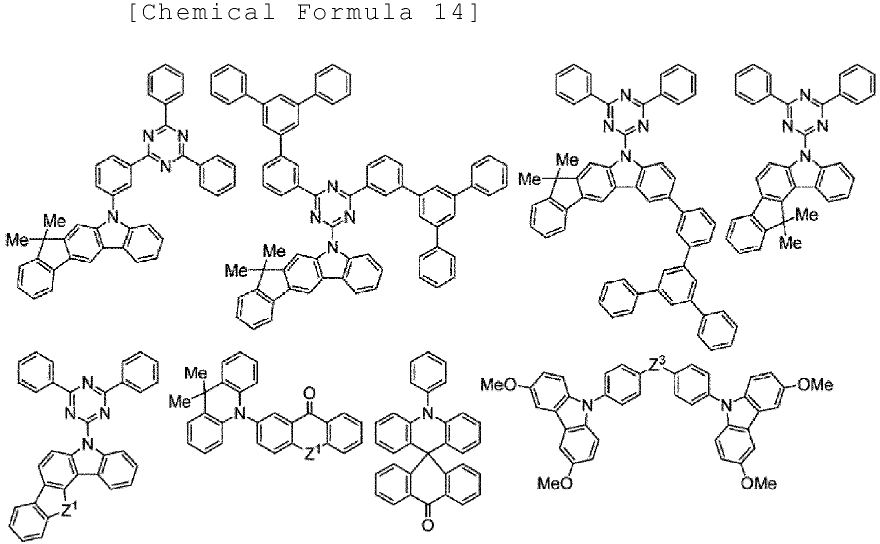

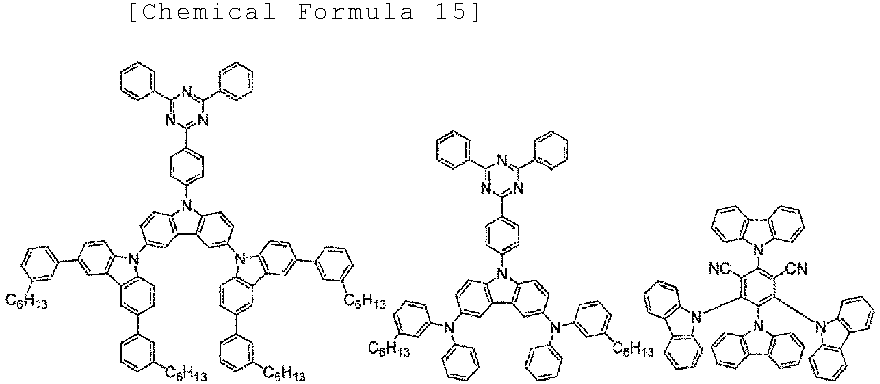

### [Host material]

It is preferable that the composition for light emitting device of the present embodiment further contains a host material having at least one function selected from hole injectability, hole transportability, electron injectability and electron transportability, since the light emitting device of the present embodiment is more excellent in light emission efficiency. The composition for light emitting device of the present embodiment may contain the host material singly or in combination of two or more. However, the host material is different from the compound (B). Further, the host material is different from the compound (A).

When the composition for light emitting device of the present embodiment further contains a host material, the host material, the compound (B) and the compound (A) preferably interact physically, chemically or electrically. By this interaction, it becomes possible to improve or adjust, for example, the light emitting property, the charge transporting property or the charge injection property of the composition for light emitting device of the present embodiment.

When the composition for light emitting device of the present embodiment further contains a host material, if a light emitting material is explained as an example, the host material, the compound (B) and the compound (A) interact electrically to transfer electrical energy efficiently from the host material to the compound (B) and further to transfer electrical energy efficiently from the compound (B) to the compound (A), accordingly, the compound (A) can be allowed to emit light more efficiently, and the light emitting device of the present embodiment is more excellent in light emission efficiency.

From the above-described standpoint, when the composition for light emitting device of the present embodiment further contains a host material, it is preferable that the lowest excited singlet state (Si) of the host material is at the energy level higher than that of the lowest excited singlet state (Si) of the compound (A) and the compound (B), since the light emitting device of the present embodiment is more excellent in light emission efficiency.

When the composition for light emitting device of the present embodiment further contains a host material, the absolute value (hereinafter, referred to also as "|EH-AB|") of a difference between EH

(the energy value of the maximum peak of the emission spectrum at 25°C of the host material) and AB (the energy value of a peak at the lowest energy side of the absorption spectrum at 25°C of the compound (B)) is preferably 0.60 eV or less, more preferably 0.55 eV or less, and further preferably 0.50 eV or less, since the host material and the compound (B) more efficiently interact physically, chemically or electrically and the light emitting device of the present embodiment is more excellent in light emission efficiency. Meanwhile, |EH-AB| may be 0 eV or more, may be 0.001 eV or more, may be 0.005 eV or more, may be 0.01 eV or more, may be 0.05 eV or more, may be 0.10 eV or more, may be 0.20 eV or more, may be 0.30 eV or more and may be 0.35 eV or more.

EH is preferably 2.0 eV or more, and may be 2.2 eV or more, may be 2.4 eV or more, may be 2.6 eV or more, may be 2.8 eV or more and may be 3.0 eV or more. Meanwhile, EH is preferably 4.5 eV or less, more preferably 4.0 eV or less, and may be 3.8 eV or less, may be 3.6 eV or less and may be 3.4 eV or less.

AB is preferably 1.8 eV or more, and may be 2.0 eV or more, may be 2.2 eV or more, may be 2.4 eV or more, may be 2.6 eV or more and may be 2.8 eV or more. Meanwhile, AB is preferably 4.5 eV or less, more preferably 4.0 eV or less, and may be 3.8 eV or less, may be 3.6 eV or less, may be 3.4 eV or less, may be 3.2 eV or less and may be 3.0 eV or less.

Under the above-mentioned conditions, the relationship between |EH-AB| and the light emitting properties (particularly, light emission efficiency) of the light emitting device is estimated as follows.

The present inventors investigated designs by which the host material and the compound (B) efficiently interact physically, chemically or electrically (particularly, a design for efficient electrical interaction). The present inventors estimated first that the compound (B) is a compound having the small half-value width of the emission spectrum, accordingly, the half-value width of the absorption spectrum of the compound (B) is small. Then, it was estimated that when the half-value width of the absorption spectrum of the compound (B) is small, the overlap between the emission spectrum of the host material and the absorption spectrum of the compound (B) tends to be smaller. Under this state, the present inventors believed that an electrical interaction can be obtained more efficiently, by increasing the overlap between the emission spectrum of the host material and the absorption spectrum of the compound (B), and focused on |EH-AB|. More specifically, it is presumed that by regulating |EH-AB| to 0.60 eV or less, the overlap between the emission spectrum of the host material and the absorption spectrum of the compound (B) becomes large, thus, the electrical energy of the host material transfers quickly to the compound (B), and resultantly, the light emitting device is more excellent in light emitting properties (particularly, light emission efficiency).

When the composition for light emitting device of the present embodiment further contains a host material, it is preferable that |EB-AA| is 0.60 eV or less and |EH-AB| is 0.60 eV or less, since the host material, the compound (B) and the compound (A) more efficiently interact physically, chemically or electrically and the light emitting device of the present embodiment is more excellent in light emission efficiency. The examples and preferable ranges of |EB-AA| and |EH-AB| in this case are the same as the examples and preferable ranges described in the sections of |EB-AA| and |EH-AB|, respectively.

Based on the ideas described in "the relationship between |EB-AA| and the light emitting properties (particularly, light emission efficiency) of the light emitting device" described above and "the relationship between |EH-AB| and the light emitting properties (particularly, light emission efficiency) of the light emitting device" described above, it is presumed that when the composition for light emitting device of the present embodiment further contains a host material, if |EB-AA| is regulated to 0.60 eV or less, the overlap between the emission spectrum of the host material and the absorption spectrum of the compound (B) increases and the electrical energy of the host material transfers quickly to the compound (B), and further, if |EH-AB| is regulated to 0.60 eV or less, the overlap between the emission spectrum of the compound (B) and the absorption spectrum of the compound (A) increases and the electrical energy of the compound (B) transfers quickly to the compound (A), accordingly, the compound (A) can be allowed to emit light more efficiently, and resultantly, the light emitting device is more excellent in the light emitting properties (particularly, light emission efficiency).

When the composition for light emitting device of the present embodiment further contains a host material, it is preferable that EH>AB is satisfied, since the light emitting device of the present embodiment is more excellent in light emission efficiency.

When the composition for light emitting device of the present embodiment further contains a host material, it is preferable that EH>EB is satisfied, since the light emitting device of the present embodiment is more excellent in light emission efficiency.

When the composition for light emitting device of the present embodiment further contains a host material, it is preferable that EH>EA is satisfied, since the light emitting device of the present embodiment is more excellent in light emission efficiency.

When the composition for light emitting device of the present embodiment further contains a host material, the content of the host material is usually 1 to 99.99 parts by mass, preferably 10 to 99.9 parts by mass, more preferably 30 to 99.5 parts by mass, further preferably 50 to 99 parts by mass, particularly preferably 70 to 98 parts by mass, especially preferably 80 to 97 parts by mass, and may be 90 to 97 parts by mass, when the sum of the compound (A), the compound (B) and the host material is taken as 100 parts by mass.

The host material is preferably one showing solubility in a solvent which is capable of dissolving the compound (A) and the compound (B), since the light emitting device of the present embodiment can be fabricated by a wet method.

The host material is classified into low molecular weight compounds (low molecular weight host) and polymer compounds (polymer host), and the composition for light emitting device of the present embodiment may contain any host material. The host material which may be contained in the composition for light emitting device of the present embodiment is preferably a low molecular weight compound, since the light emitting device of the present embodiment is more excellent in light emission efficiency.

The polymer host includes, for example, polymer compounds as a hole transporting material described later and polymer compounds as an electron transporting material described later.

The low molecular weight host is preferably a compound represented by the formula (H-1), since the light emitting device of the present embodiment is more excellent in light emission efficiency. The compound represented by the formula (H-1) is preferably a compound having no condensed hetero ring skeleton (b) in the compound.

The molecular weight of the compound represented by the formula (H-1) is preferably 1×10² to 5×10³, more preferably 2×10² to 3×10³, further preferably 3×10² to 1.5×10³, and particularly preferably 4×10² to 1×10³.

The aryl group represented by Ar^{H1} and Ar^{H2} is preferably a group obtained by removing from a monocyclic or di to heptacyclic aromatic hydrocarbon one hydrogen atom bonding directly to an atom constituting the ring, more preferably a group obtained by removing from a monocyclic or di to pentacyclic aromatic hydrocarbon one hydrogen atom bonding directly to an atom constituting the ring, further preferably a group obtained by removing from benzene, naphthalene, anthracene, phenanthrene, dihydrophenanthrene, fluorene, benzoanthracene, benzophenanthrene, benzofluorene, pyrene, fluoranthene, perylene or benzofluoranthene one hydrogen atom bonding directly to an atom constituting the ring, and particularly preferably a group obtained by removing from benzene, naphthalene, anthracene, fluorene, pyrene or benzofluoranthene one hydrogen atom bonding directly to an atom constituting the ring, and these groups optionally have a substituent.

The arylene group represented by L^{H1} is preferably a group obtained by removing from a monocyclic or di to heptacyclic aromatic hydrocarbon two hydrogen atoms bonding directly to atoms constituting the ring, more preferably a group obtained by removing from a monocyclic or di to pentacyclic aromatic hydrocarbon two hydrogen atoms bonding directly to atoms constituting the ring, further preferably a group obtained by removing from benzene, naphthalene, anthracene, phenanthrene, dihydrophenanthrene, fluorene, benzoanthracene, benzophenanthrene, benzofluorene, pyrene, fluoranthene, perylene or benzofluoranthene two hydrogen atoms bonding directly to atoms constituting the ring, and particularly preferably a group obtained by removing from benzene, naphthalene, anthracene, fluorene, pyrene or benzofluoranthene two hydrogen atoms bonding directly to atoms constituting the ring, and these groups optionally have a substituent.

The monovalent hetero ring group represented by Ar^{H1} and Ar^{H2} is preferably a group obtained by removing from a heterocyclic compound containing no condensed hetero ring skeleton (b) one hydrogen atom bonding directly to an atom constituting the ring, and this group optionally has a substituent. In the monovalent hetero ring group represented by Ar^{H1} and Ar^{H2}, the heterocyclic compound containing no condensed hetero ring skeleton (b) includes heterocyclic compounds not containing a boron atom and a nitrogen atom in the ring among heterocyclic compounds described in the section of the hetero ring group described above. The monovalent hetero ring group represented by Ar^{H1} and Ar^{H2} is preferably a group obtained by removing from a monocyclic or di to heptacyclic heterocyclic compound (preferably, a monocyclic or di to heptacyclic heterocyclic compound containing no condensed hetero ring skeleton (b)) one hydrogen atom bonding directly to an atom constituting the ring, more preferably a group obtained by removing from a monocyclic or di to pentacyclic heterocyclic compound (preferably, a monocyclic or di to pentacyclic heterocyclic compound containing no condensed hetero ring skeleton (b)) one hydrogen atom bonding directly to an atom constituting the ring, further preferably a group obtained by removing from pyridine, diazabenzene, triazine, azanaphthalene, diazanaphthalene, dibenzofuran, dibenzothiophene, carbazole, phenoxazine, phenothiazine, benzocarbazole, benzonaphthofuran, benzonaphthothiophene, dibenzocarbazole, indolocarbazole or indenocarbazole one hydrogen atom bonding directly to an atom constituting the ring, and particularly preferably a group obtained by removing from pyridine, diazabenzene, triazine, azanaphthalene, diazanaphthalene, dibenzofuran, dibenzothiophene, carbazole, benzocarbazole, benzonaphthofuran or benzonaphthothiophene one hydrogen atom bonding directly to an atom constituting the ring, and these groups optionally have a substituent.

The divalent hetero ring group represented by L^{H1} is preferably a group obtained by removing from a heterocyclic compound containing no condensed hetero ring skeleton (b) two hydrogen atoms bonding directly to atoms constituting the ring. In the divalent hetero ring group represented by L^{H1}, the heterocyclic compound containing no condensed hetero ring skeleton (b) includes heterocyclic compounds not containing a boron atom and a nitrogen atom in the ring among heterocyclic compounds described in the section of the hetero ring group described above. The divalent hetero ring group represented by L^{H1} is preferably a group obtained by removing from a monocyclic or di to heptacyclic heterocyclic compound (preferably, a monocyclic or di to heptacyclic heterocyclic compound containing no condensed hetero ring skeleton (b)) two hydrogen atoms bonding directly to atoms constituting the ring, more preferably a group obtained by removing from a monocyclic or di to pentacyclic heterocyclic compound (preferably, a monocyclic or di to pentacyclic heterocyclic compound containing no condensed hetero ring skeleton (b)) two hydrogen atoms bonding directly to atoms constituting the ring, further preferably a group obtained by removing from pyridine, diazabenzene, triazine, azanaphthalene, diazanaphthalene, dibenzofuran, dibenzothiophene, carbazole, phenoxazine, phenothiazine, benzocarbazole, benzonaphthofuran, benzonaphthothiophene, dibenzocarbazole, indolocarbazole or indenocarbazole two hydrogen atoms bonding directly to atoms constituting the ring, and particularly preferably a group obtained by removing from pyridine, diazabenzene, triazine, azanaphthalene, diazanaphthalene, dibenzofuran, dibenzothiophene, carbazole, benzocarbazole, benzonaphthofuran or benzonaphthothiophene two hydrogen atoms bonding directly to atoms constituting the ring, and these groups optionally have a substituent.

In the substituted amino group represented by Ar^{H1} and Ar^{H2}, the substituent which the amino group has is preferably an aryl group or a monovalent hetero ring group, and more preferably an aryl group, and these groups optionally further have a substituent. The examples and preferable ranges of the aryl group as the substituent which the amino group has are the same as the examples and preferable ranges of the aryl group represented by Ar^{H1} and Ar^{H2}. The examples and preferable ranges of the monovalent hetero ring group as the substituent which the amino group has are the same as the examples and preferable ranges of the monovalent hetero ring group represented by Ar^{H1} and Ar^{H2}.

It is preferable that at least one of Ar^{H1} and Ar^{H2} is an aryl group or a monovalent hetero ring group, it is more preferable that both Ar^{H1} and Ar^{H2} are aryl groups or monovalent hetero ring groups, and it is further preferable that both Ar^{H1} and Ar^{H2} are monovalent hetero ring groups, since the light emitting device of the present embodiment is more excellent in light emission efficiency, and these groups optionally have a substituent.

The aryl group and the monovalent hetero ring group represented by Ar^{H1} and Ar^{H2} are each preferably a group obtained by removing from benzene, naphthalene, fluorene, pyridine, diazabenzene, triazine, azanaphthalene, diazanaphthalene, dibenzofuran, dibenzothiophene or carbazole one hydrogen atom bonding directly to an atom constituting the ring, more preferably a phenyl group, a naphthyl group, a fluorenyl group, a carbazolyl group, a dibenzothienyl group or a dibenzofuryl group, and further preferably a phenyl group, a naphthyl group or a carbazolyl group, since the light emitting device of the present embodiment is more excellent in light emission efficiency, and these groups optionally have a substituent.

It is preferable that at least one of L^{H1} is an arylene group or a divalent hetero ring group, it is more preferable that all of L^{H1} are arylene groups or divalent hetero ring groups, and it is further preferable that all of L^{H1} are divalent hetero ring groups, since the light emitting device of the present embodiment is more excellent in light emission efficiency, and these groups optionally have a substituent.

The arylene group and the divalent hetero ring group represented by L^{H1} are each preferably a group obtained by removing from benzene, naphthalene, anthracene, fluorene, pyrene, benzofluoranthene, pyridine, diazabenzene, triazine, azanaphthalene, diazanaphthalene, dibenzofuran, dibenzothiophene or carbazole two hydrogen atoms bonding directly to atoms (preferably, carbon atoms) constituting the ring, more preferably a group obtained by removing from benzene, naphthalene, anthracene, dibenzofuran, dibenzothiophene or carbazole two hydrogen atoms bonding directly to atoms (preferably, carbon atoms) constituting the ring, and further preferably a group obtained by removing from benzene, naphthalene, anthracene, dibenzofuran or dibenzothiophene two hydrogen atoms bonding directly to atoms constituting the ring, since the light emitting device of the present embodiment is more excellent in light emission efficiency, and these groups optionally have a substituent.

The substituent which Ar^{H1}, Ar^{H2} and L^{H1} optionally have is preferably an alkyl group, a cycloalkyl group, an alkoxy group, a cycloalkoxy group, an aryl group, a monovalent hetero ring group, a substituted amino group, a cyano group or halogen atom, more preferably an alkyl group, a cycloalkyl group, an aryl group, a monovalent hetero ring group or a substituted amino group, and further preferably an alkyl group, an aryl group or a monovalent hetero ring group, and these groups optionally further have a substituent.

The examples and preferable ranges of the aryl group, the monovalent hetero ring group and the substituted amino group as the substituent which Ar^{H1}, Ar^{H2} and L^{H1} optionally have are the same as the examples and preferable ranges of the aryl group, the monovalent hetero ring group and the substituted amino group represented by Ar^{H1} and Ar^{H2}, respectively.

The substituent which the substituent which Ar^{H1}, Ar^{H2} and L^{H1} optionally have optionally further has is preferably an alkyl group, a cycloalkyl group, an aryl group, a monovalent hetero ring group or a substituted amino group, and more preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which Ar^{H1}, Ar^{H2} and L^{H 1} optionally have optionally further has are the same as the examples and preferable ranges of the aryl group, the monovalent hetero ring group and the substituted amino group represented by Ar^{H1} and Ar^{H2}, respectively.

n^{H 1} is usually an integer of 0 or more and 10 or less, preferably an integer of 0 or more and 7 or less, more preferably an integer of 1 or more and 5 or less, further preferably an integer of 1 or more and 3 or less, and particularly preferably 1.

The compound represented by the formula (H-1) includes, for example, compounds represented by the following formulae. In the formulae, Z¹ represents an oxygen atom or a sulfur atom. In the formulae, Z² represents a group represented by -CH= or a group represented by -N=.

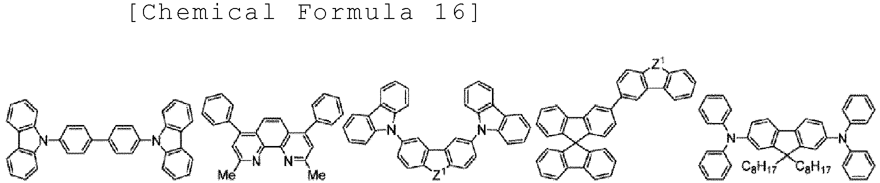

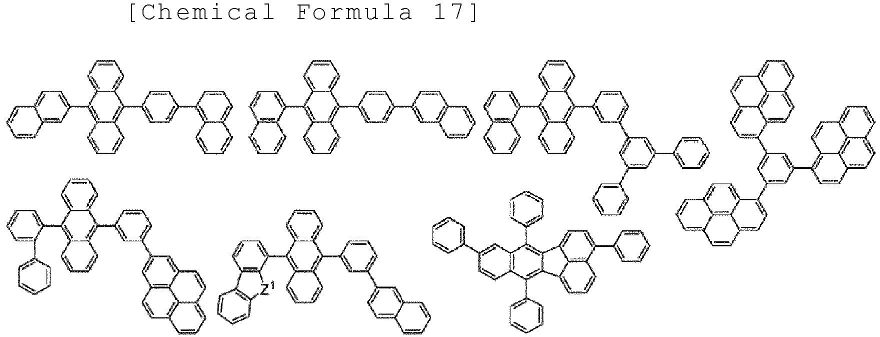

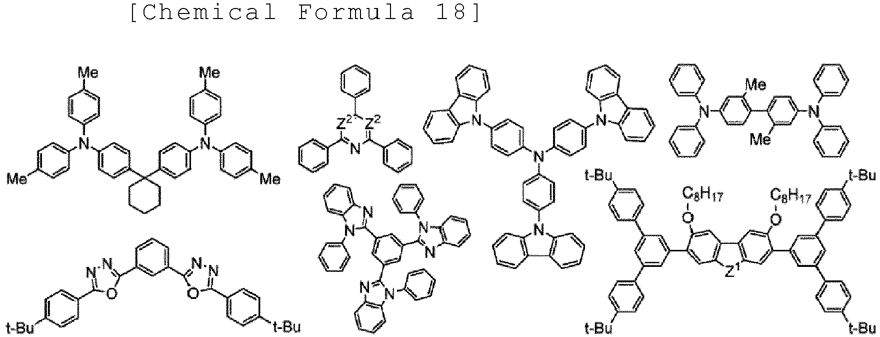

### [Other components]

The composition for light emitting device of the present embodiment may be a composition containing the compound (A) and the compound (B), and at least one selected from the group consisting of a host material, a hole transporting material, a hole injection material, an electron transporting material, an electron injection material, a light emitting material, an antioxidant and a solvent described above. The hole transporting material, the hole injection material, the electron transporting material, the electron injection material and the light emitting material are different from the compound (A) and the compound (B).

### [Ink]

The composition containing the compound (A) and the compound (B), and a solvent (hereinafter, referred to as "ink") is suitable for fabricating a light emitting device using a wet method such as, for example, a spin coat method, a casting method, a microgravure coat method, a gravure coat method, a bar coat method, a roll coat method, a wire bar coat method, a dip coat method, a spray coat method, a screen printing method, a flexo printing method, an offset printing method, an inkjet printing method, a capillary coat method, a nozzle coat method and the like. The viscosity of the ink may be adjusted according to the type of the printing method, and is preferably 1 mPa•s to 20 mPa•s at 25°C.

The solvent contained in the ink is preferably a solvent capable of dissolving or uniformly dispersing solid components in the ink. The solvent includes, for example, chlorine-based solvents, ether-based solvents, aromatic hydrocarbon-based solvents, aliphatic hydrocarbon-based solvents, ketone-based solvents, ester-based solvents, polyhydric alcohol-based solvents, alcohol-based solvents, sulfoxide-based solvents and amide-based solvents.

In the ink, the compounding amount of the solvent is usually 1000 parts by mass to 10000000 parts by mass, when the sum of the compound (A) and the compound (B) is taken as 100 parts by mass.

The solvent may be used singly or in combination of two or more.

### • Hole transporting material

The hole transporting material is classified into low molecular weight compounds and polymer compounds, and polymer compounds having a cross-linkable group are preferable.

The polymer compound includes, for example, polyvinylcarbazole and derivatives thereof; and polyarylenes having an aromatic amine structure in the side chain or main chain, and derivatives thereof. The polymer compound may be a compound to which an electron accepting site such as fullerene, tetrafluorotetracyanoquinodimethane, tetracyanoethylene, trinitrofluorenone and the like is bonded.

In the composition for light emitting device of the present embodiment, when a hole transporting material is contained, the compounding amount of the hole transporting material is usually 1 part by mass to 10000 parts by mass, when the sum of the compound (A) and the compound (B) is taken as 100 parts by mass.

The hole transporting material may be used singly or in combination of two or more.

### • Electron transporting material

The electron transporting material is classified into low molecular weight compounds and polymer compounds. The electron transporting material may have a cross-linkable group.

The low molecular weight compound includes, for example, a metal complex having 8-hydroxyquinoline as a ligand, oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the composition for light emitting device of the present embodiment, when an electron transporting material is contained, the compounding amount of the electron transporting material is usually 1 part by mass to 10000 parts by mass, when the sum of the compound (A) and the compound (B) is taken as 100 parts by mass.

The electron transporting material may be used singly or in combination of two or more.

### • Hole injection material and electron injection material

The hole injection material and the electron injection material are each classified into low molecular weight compounds and polymer compounds. The hole injection material and the electron injection material may have a cross-linkable group.

The low molecular weight compound includes, for example, metal phthalocyanines such as copper phthalocyanine and the like; carbon; oxides of metals such as molybdenum, tungsten and the like; metal fluorides such as lithium fluoride, sodium fluoride, cesium fluoride, potassium fluoride and the like.

The polymer compound includes electrically conductive polymers such as, for example, polyaniline, polythiophene, polypyrrole, polyphenylenevinylene, polythienylenevinylene, polyquinoline and polyquinoxaline, and derivatives thereof; a polymer containing an aromatic amine structure in the main chain or side chain, and the like.

In the composition for light emitting device of the present embodiment, when a hole injection material and/or an electron injection material is contained, the compounding amounts of the hole injection material and the electron injection material are each usually 1 part by mass to 10000 parts by mass, when the sum of the compound (A) and the compound (B) is taken as 100 parts by mass.

The hole injection material and the electron injection material each may be used singly or in combination of two or more.

### • Ion doping

When the hole injection material or the electron injection material contains an electrically conductive polymer, the electric conductivity of the electrically conductive polymer is preferably 1×10⁻⁵ S/cm to 1×10³ S/cm. For adjusting the electric conductivity of the electrically conductive polymer within such a range, the electrically conductive polymer can be doped with an appropriate amount of ions. The kind of the ion to be doped is an anion for the hole injection material and a cation for the electron injection material. The anion includes, for example, a polystyrenesulfonic ion, an alkylbenzenesulfonic ion and a camphorsulfonic ion. The cation includes, for example, a lithium ion, a sodium ion, a potassium ion and a tetrabutylammonium ion.

The ions to be doped may be used singly or in combination of two or more.

### • Light emitting material

The light emitting material is classified into low molecular weight compounds and polymer compounds. The light emitting material may have a cross-linkable group.

The low molecular weight compound includes, for example, naphthalene and derivatives thereof, anthracene and derivatives thereof, perylene and derivatives thereof, and triplet light emitting complexes having iridium, platinum or europium as the central metal.

The polymer compound includes polymer compounds containing, for example, an arylene group such as a phenylene group, a naphthalenediyl group, a fluorenediyl group, a phenanthrenediyl group, a dihydrophenanthrenediyl group, an anthracenediyl group, a pyrenediyl group and the like; an aromatic amine residue such as a group obtained by removing from an aromatic amine two hydrogen atoms, and the like; and a divalent hetero ring group such as a carbazolediyl group, a phenoxazinediyl group, a phenothiazinediyl group and the like.

In the composition for light emitting device of the present embodiment, when a light emitting material is contained, the content of the light emitting material is usually 0.1 parts by mass to 10000 parts by mass, when the sum of the compound (A) and the compound (B) is taken as 100 parts by mass.

The light emitting material may be used singly or in combination of two or more.

### • Antioxidant

The antioxidant may a compound which is soluble in the same solvent as for the compound (A) and the compound (B) and does not inhibit light emission and charge transportation, and includes, for example, phenol type antioxidants and phosphorus-based antioxidants.

In the composition for light emitting device of the present embodiment, when an antioxidant is contained, the compounding amount of the antioxidant is usually 0.00001 parts by mass to 10 parts by mass, when the sum of the compound (A) and the compound (B) is taken as 100 parts by mass.

The antioxidant may be used singly or in combination of two or more.

### <Film>

The film of the present embodiment contains the composition for light emitting device described above. The film of the present embodiment is suitable as a light emitting layer in a light emitting device. The film of the present embodiment can be fabricated, for example, by a wet method using an ink. Further, the film of the present embodiment can be fabricated, for example, by a dry method such as a vacuum vapor deposition method and the like. The method for fabricating the film of the present embodiment by a dry method includes, for example, a method of vapor-depositing the above-described composition for light emitting device and a method of co-vapor-depositing the compound (A) and the compound (B).

The thickness of the film is usually 1 nm to 10 µm.

### <Light emitting device>

The light emitting device of the present embodiment contains the composition for light emitting device described above.

The light emitting device of the present embodiment may be one having, for example, an anode, a cathode, and an organic layer containing the above-described composition for light emitting device disposed between the anode and the cathode.

### [Layer constitution]

The layer containing the composition for light emitting device of the present embodiment is usually one or more layers selected from the group consisting of a light emitting layer, a hole transporting layer, a hole injection layer, an electron transporting layer and an electron injection layer, and preferably is a light emitting layer. These layers contain a light emitting material, a hole transporting material, a hole injection material, an electron transporting material and an electron injection material, respectively. These layers can be formed by the same method as for the fabrication of the film described above using a light emitting material, a hole transporting material, a hole injection material, an electron transporting material and an electron injection material, respectively.

The light emitting device has a light emitting layer between an anode and a cathode. The light emitting device of the present embodiment preferably has at least one of a hole injection layer and a hole transporting layer between an anode and a light emitting layer, from the standpoint of hole injectability and hole transportability, and preferably has at least one of an electron injection layer and an electron transporting layer between a cathode and a light emitting layer, from the standpoint of electron injectability and electron transportability.

The materials of a hole transporting layer, an electron transporting layer, a light emitting layer, a hole injection layer and an electron injection layer include the hole transporting material, the electron transporting material, the light emitting material, the hole injection material and the electron injection material and the like described above, respectively, in addition to the composition for light emitting device of the present embodiment.

When the material of a hole transporting layer, the material of an electron transporting layer and the material of a light emitting layer are soluble in a solvent used in forming layers adjacent to the hole transporting layer, the electron transporting layer and the light emitting layer, respectively, in fabricating a light emitting device, it is preferable that the material has a cross-linkable group for avoiding the material from being dissolved in the solvent. After forming each layer using the material having a cross-linkable group, the cross-linkable group can be cross-linked to insolubilize the layer.

The method for forming each layer such as a light emitting layer, a hole transporting layer, an electron transporting layer, a hole injection layer, an electron injection layer and the like in the light emitting device of the present invention includes, for example, dry methods such as a method of vacuum vapor-deposition from a powder and the like and wet methods such as a method by film formation from a solution or melted state and the like when a low molecular weight compound is used, and includes, for example, wet methods such as a method by film formation from a solution or melted state and the like when a polymer compound is used. The order, number and thickness of layers to be laminated are adjusted in consideration of light emission efficiency, driving voltage and luminance life.

### [Substrate/electrode]

The substrate of a light emitting device may be a substrate on which an electrode can be formed and which does not chemically change in forming an organic layer, and it is, for example, a substrate made of a material such as glass, plastic, silicon and the like. In the case of an opaque substrate, it is preferable that the electrode farthest from the substrate is transparent or semi-transparent.

The material of the anode includes, for example, electrically conductive metal oxides and semi-transparent metals, preferably includes indium oxide, zinc oxide, tin oxide; electrically conductive compounds such as indium-tin-oxide (ITO), indium-zinc-oxide and the like; argentine-palladium-copper (APC) complex; NESA, gold, platinum, silver and copper.

The material of the cathode includes, for example, metals such as lithium, sodium, potassium, rubidium, cesium, beryllium, magnesium, calcium, strontium, barium, aluminum, zinc, indium and the like; alloys composed of two or more of them; alloys composed of one or more of them and one or more of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

The anode and the cathode each may take a laminated structure composed of two or more layers.

### [Application]

The light emitting device of the present embodiment can be suitably used as a light source for backlight of a liquid crystal display device, a light source for illumination, organic EL illumination, a display device of computers, televisions, portable terminals and the like (for example, organic EL display and organic EL television).

Suitable embodiments of the present invention have been explained above, but the present invention is not limited to them.

For example, one aspect of the present invention may relate to a method for producing the composition for light emitting device described above.

In one embodiment, the method for producing a composition for light emitting device may be a production method comprising a preparation step of preparing a thermally activated delayed fluorescent compound (A), a sorting step of sorting a compound (B) which is a compound having a condensed hetero ring skeleton (b) and in which the energy value (EB) of the maximum peak of the emission spectrum at 25°C shows a value with which the difference (EB-AA) from the energy value (AA) of a peak at the lowest energy side of the absorption spectrum at 25°C of the compound (A) is 0.60 eV or less, and a production step of mixing the compound (A) prepared in the preparation step and the compound (B) sorted in the sorting step to obtain a composition for light emitting device (hereinafter, referred to also as "production method (1)").

The production method (1) may further comprise a step of determining EB (the energy value of the maximum peak of the emission spectrum at 25°C of the compound (B)). This step of determining EB may be included in the sorting step, in the production method (1).

The production method (1) may further comprise a step of determining AA (the energy value of a peak at the lowest energy side of the absorption spectrum at 25°C of the compound (A)). This step of determining AA may be included in the preparation step or the sorting step, or may be further included in the sorting step, in the production method (1).

The production method (1) may further comprise a step of determining EB and AA and calculating the absolute value (|EB-AA|) of a difference between them. This step of calculating |EB-AA| may be included in the sorting step, in the production method (1).

The production method (1) may further comprise a step of determining each of the energy level of the lowest triplet excited state and the energy level of the lowest singlet excited state of the compound (B) and calculating the absolute value ΔE_{ST} of a difference between them. This step of calculating ΔE_{ST} may be included in the sorting step, in the production method (1).

The production method (1) may further comprise a step of determining each of the energy level of the lowest triplet excited state and the energy level of the lowest singlet excited state of the compound (A) and calculating the absolute value ΔE_{ST} of a difference between then. This step of calculating ΔE_{ST} may be included in the preparation step or the sorting step, in the production method (1).

In the production method (1), it is preferable to prepare a compound (A) in which ΔE_{ST} is 0.50 eV or less, in the preparation step.

Further, in the production method (1), it is preferable to sort a compound (B) in which ΔE_{ST} is 0.50 eV or less, in the sorting step.

In the sorting step in the production method (1), the compound (B) may be further sorted such that ΔE_{ST} of the compound (B) is larger than ΔE_{ST} of the compound (A).

In the production method (1), the production step may be a step of mixing the compound (A) prepared in the preparation step, the compound (B) sorted in the sorting step and the host material. According to the production method (hereinafter, referred to also as "production method (1')") as described above, a composition for light emitting device containing the fluorescent compound prepared in the preparation step, the compound (B) sorted in the sorting step and the host material is obtained.

In the production method (1'), the production step may be a step of mixing the compound (A) prepared in the preparation step, the compound (B) sorted in the sorting step and the host material simultaneously, or may be a step of mixing the host material with a mixture composed of the compound (A) prepared in the preparation step and the compound (B) sorted in the sorting step.

In the production method (1'), the compound (B) may be further sorted in the sorting step such that the absolute value (|EH-AB|) of a difference between the energy value (EH) of the maximum peak of the emission spectrum at 25°C of the host material and the energy value (AB) of a peak at the lowest energy side of the absorption spectrum at 25°C of the compound (B) is 0.60 eV or less.

The production method (1') may further comprise a host material sorting step of sorting the host material such that the absolute value (|EH-AB|) of a difference between the energy value (EH) of the maximum peak of the emission spectrum at 25°C of the host material and the energy value (AB) of a peak at the lowest energy side of the absorption spectrum at 25°C of the compound (B) sorted in the sorting step is 0.60 eV or less. In this case, the compound (A) prepared in the preparation step, the compound (B) sorted in the sorting step and the host material sorted in the host material sorting step are mixed in the production step.

The production method (1') may further comprise a step of determining EH (the energy value of the maximum peak of the emission spectrum at 25°C of the host material). This step of determining EH may be included in the sorting step or the host material sorting step.

The production method (1') may further comprise a step of determining AB (the energy value of a peak at the lowest energy side of the absorption spectrum at 25°C of the compound (B)). This step of determining AB may be included in the sorting step or the host material sorting step.

In another embodiment, the method for producing a composition for light emitting device may be a production method comprising a preparation step of preparing a compound (B) having a condensed hetero ring skeleton (b), a sorting step of sorting a thermally activated delayed fluorescent compound (A) in which the energy value (AA) of a peak at the lowest energy side of the absorption spectrum at 25°C shows value with which the difference (EB-AA) from the energy value (EB) of the maximum peak of the emission spectrum at 25°C of the compound (B) is 0.60 eV or less, and a production step of mixing the compound (B) prepared in the preparation step and the compound (A) sorted in the sorting step to obtain a composition for light emitting device (hereinafter, referred to also as "production method (2)").

The production method (2) may further comprise a step of determining EB (the energy value of the maximum peak of the emission spectrum at 25°C of the compound (B)). This step of determining EB may be included in the preparation step or the sorting step, or may be further included in the sorting step, in the production method (2).

The production method (2) may further comprise a step of determining AA (the energy value of a peak at the lowest energy side of the absorption spectrum at 25°C of the compound (A)). This step of determining AA may be included in the sorting step, in the production method (2).

The production method (2) may further comprise a step of determining EB and AA and calculating the absolute value (|EB-AA|) of a difference between them. This step of calculating |EB-AA| may be included in the sorting step, in the production method (2).

The production method (2) may further comprise a step of determining each of the energy level of the lowest triplet excited state and the energy level of the lowest singlet excited state of the compound (B) and calculating the absolute value ΔE_{ST} of a difference between them. This step of calculating ΔE_{ST} may be included in the preparation step or the sorting step, in the production method (2).

The production method (2) may further comprise a step of determining each of the energy level of the lowest triplet excited state and the energy level of the lowest singlet excited state of the compound (A) and calculating the absolute value ΔE_{ST} of a difference between them. This step of calculating ΔE_{ST} may be included in the sorting step, in the production method (2).

In the production method (2), it is preferable to prepare a compound (B) in which ΔE_{ST} is 0.50 eV or less, in the preparation step.

Further, in the production method (2), it is preferable to sort a compound (A) in which ΔE_{ST} is 0.50 eV or less, in the sorting step.

In the sorting step in the production method (2), the compound (A) may be further sorted such that ΔE_{ST} of the compound (A) is smaller than ΔE_{ST} of the compound (B).

In the production method (2), the production step may be a step of mixing the compound (B) prepared in the preparation step, the compound (A) sorted in the sorting step and the host material. According to the production method (hereinafter, referred to also as "production method (2')") as described above, a composition for light emitting device containing the compound (B) prepared in the preparation step, the compound (A) sorted in the sorting step and the host material is obtained.

In the production method (2'), the production step may be a step of mixing the compound (B) prepared in the preparation step, the compound (A) sorted in the sorting step and the host material simultaneously, or may be a step of mixing the host material with a mixture composed of the compound (B) prepared in the preparation step and the compound (A) sorted in the sorting step.

The production method (2') may further comprise a host material preparation step. In this case, the preparation step (a step of preparing a compound (B)) may be a step of preparing a compound (B) in which the absolute value (|EH-AB|) of a difference between the energy value (EH) of the maximum peak of the emission spectrum at 25°C of the host material and the energy value (AB) of a peak at the lowest energy side of the absorption spectrum at 25°C of the compound (B) is 0.60 eV or less.

The production method (2') may further comprise a host material sorting step. This host material sorting step may be a step of sorting a host material in which the absolute value (|EH-AB|) of a difference between the energy value (EH) of the maximum peak of the emission spectrum at 25°C of the host material and the energy value (AB) of a peak at the lowest energy side of the absorption spectrum at 25°C of the compound (B) prepared in the preparation step is 0.60 eV or less. In this case, the compound (B) prepared in the preparation step, the compound (A) sorted in the sorting step and the host material sorted in the host material sorting step are mixed, in the production step.

The production method (2') may further comprise a step of determining EH (the energy value of the maximum peak of the emission spectrum at 25°C of the host material). This step of determining EH may be included in the host material preparation step, the preparation step (a step of preparing a compound (B)) or the host material sorting step.

The production method (2') may further comprise a step of determining AB (the energy value of a peak at the lowest energy side of the absorption spectrum at 25°C of the compound (B)). This step of determining AB may be included in the preparation step (a step of preparing a compound (B)) or the host material sorting step.

In the production steps in the production method (1) and the production method (2), the method of mixing a compound (A) and a compound (B) is not particularly restricted. The mixing method includes, for example, a method of dissolving a compound (A) and a compound (B) in a solvent described in the section of the ink described above and mixing them, a method of mixing a compound (A) and a compound (B) in the solid state, a method of mixing a compound (A) and a compound (B) by co-vapor-deposition, and the like.

In the production steps in the production method (1') and the production method (2'), the method of mixing a compound (A), a compound (B) and a host material is not particularly restricted. The mixing method includes, for example, a method of dissolving a compound (A), a compound (B) and a host material in a solvent described in the section of the ink described above and mixing them, a method of mixing a compound (A), a compound (B) and a host material in the solid state, a method of mixing a compound (A), a compound (B) and a host material by co-vapor-deposition, and the like.

Still another aspect of the present invention may relate to the method for producing a light emitting device described above.

In one embodiment, the method for producing a light emitting device may be a production method of a light emitting device containing an anode, a cathode, and an organic layer disposed between the anode and the cathode, and this production method comprises a step of producing a composition for light emitting device by the above-described production method (for example, production method (1), production method (2), production method (1') and production method (2')) and a step of forming an organic layer using the composition for light emitting device produced by the above-described step.

In this embodiment, the organic layer can be formed using the same method as for the film fabrication described above. Further, in the method for producing a light emitting device according to this embodiment, the production method explained in the section of <Light emitting device> described above may be used. Further, the light emitting device obtained by the method for producing a light emitting device according to this embodiment includes, for example, light emitting devices explained in the section of <Light emitting device> described above.

### EXAMPLES

The present invention will be illustrated in detail by examples below, but the present invention is not limited to these examples.

For calculation of the value of ΔE_{ST} of a compound, the ground state of the compound was structurally optimized by density-functional approach at B3LYP level, and in this procedure, 6-31G* was used as the basis function. Using Gaussian09 as the quantum chemistry calculation program, ΔE_{ST} of the compound was calculated by time-dependent density-functional approach at B3LYP level.

In examples, the maximum peak wavelength of the emission spectrum of a compound at room temperature was measured by a spectrophotometer (manufactured by JASCO Corporation, FP-6500) at room temperature. A compound was dissolved in xylene at a concentration of about 8×10⁻⁴% by mass and the resultant xylene solution was used as a specimen. As the excitation light, ultraviolet (UV) light having a wavelength of 325 nm was used.

In examples, the peak wavelength at the lowest energy side of the absorption spectrum at room temperature of the compound was measured by a UV Vis spectrophotometer (manufactured by Varian, Cary 5E) at room temperature. A compound was dissolved in xylene at a concentration of about 8×10⁻⁴% by mass and the resultant xylene solution was used as a specimen.

### <Acquisition and synthesis of compounds H1, T1 to T12, B1 to B3 and E1>

A compound H1 manufactured by Luminescence Technology Corp. was used. The maximum peak wavelength of the emission spectrum at room temperature of the compound H1 was 373 nm, and its energy value (EH) was 3.32 eV.

A thermally activated delayed fluorescent compound T1 was synthesized according to a method described in International Publication WO2018/062278. The maximum peak wavelength of the emission spectrum at room temperature of the thermally activated delayed fluorescent compound T1 was 535 nm, and its energy value (EB) was 2.32 eV. The energy value of the half-value width of the maximum peak of the emission spectrum at room temperature of the thermally activated delayed fluorescent compound T1 was 0.386 eV. The peak wavelength at the lowest energy side of the absorption spectrum at room temperature of the thermally activated delayed fluorescent compound T1 was 400 nm, and its energy value (AB) was 3.10 eV. ΔE_{ST} of the thermally activated delayed fluorescent compound T1 was 0.109 eV.

A thermally activated delayed fluorescent compound T2 manufactured by Amadis Chemical was used. The maximum peak wavelength of the emission spectrum at room temperature of the thermally activated delayed fluorescent compound T2 was 524 nm, and its energy value (EA) was 2.37 eV. The peak wavelength at the lowest energy side of the absorption spectrum at room temperature of the thermally activated delayed fluorescent compound T2 was 380 nm, and its energy value (AA) was 3.26 eV. ΔE_{ST} of the thermally activated delayed fluorescent compound T2 was 0.119 eV.

A thermally activated delayed fluorescent compound T3 was synthesized with reference to a method described in International Publication WO2010/136109. The maximum peak wavelength of the emission spectrum at room temperature of the thermally activated delayed fluorescent compound T3 was 511 nm, and its energy value (EA) was 2.43 eV. The peak wavelength at the lowest energy side of the absorption spectrum at room temperature of the thermally activated delayed fluorescent compound T3 was 342 nm, and its energy value (AA) was 3.63 eV. ΔE_{ST} of the thermally activated delayed fluorescent compound T3 was 0.130 eV.

A thermally activated delayed fluorescent compound T4 was synthesized with reference to a method described in JP-A No. 2010-254676. The maximum peak wavelength of the emission spectrum at room temperature of the thermally activated delayed fluorescent compound T4 was 428 nm, and its energy value (EA) was 2.90 eV. The peak wavelength at the lowest energy side of the absorption spectrum at room temperature of the thermally activated delayed fluorescent compound T4 was 328 nm, and its energy value (AA) was 3.78 eV. ΔE_{ST} of the thermally activated delayed fluorescent compound T4 was 0.576 eV.

A thermally activated delayed fluorescent compound T5 manufactured by Luminescence Technology Corp. was used. The maximum peak wavelength of the emission spectrum at room temperature of the thermally activated delayed fluorescent compound T5 was 362 nm, and its energy value (EA) was 3.43 eV. The peak wavelength at the lowest energy side of the absorption spectrum at room temperature of the thermally activated delayed fluorescent compound T5 was 340 nm, and its energy value (AA) was 3.65 eV. Δ E_{ST} of the thermally activated delayed fluorescent compound T5 was 0.448 eV.

A thermally activated delayed fluorescent compound T6 was synthesized with reference to a method described in International Publication WO2007/063754. The maximum peak wavelength of the emission spectrum at room temperature of the thermally activated delayed fluorescent compound T6 was 470 nm, and its energy value (EA) was 2.64 eV. The peak wavelength at the lowest energy side of the absorption spectrum at room temperature of the thermally activated delayed fluorescent compound T6 was 371 nm, and its energy value (AA) was 3.34 eV. ΔE_{ST} of the thermally activated delayed fluorescent compound T6 was 0.107 eV.

A thermally activated delayed fluorescent compound T7 was synthesized with reference to a method described in International Publication WO2011/070963. The maximum peak wavelength of the emission spectrum at room temperature of the thermally activated delayed fluorescent compound T7 was 477 nm, and its energy value (EA) was 2.60 eV. The peak wavelength at the lowest energy side of the absorption spectrum at room temperature of the thermally activated delayed fluorescent compound T7 was 375 nm, and its energy value (AA) was 3.31 eV. ΔE_{ST} of the thermally activated delayed fluorescent compound T7 was 0.096 eV.

A thermally activated delayed fluorescent compound T8 manufactured by Luminescence Technology Corp. was used. The maximum peak wavelength of the emission spectrum at room temperature of the thermally activated delayed fluorescent compound T8 was 452 nm, and its energy value (EA) was 2.74 eV. The peak wavelength at the lowest energy side of the absorption spectrum at room temperature of the thermally activated delayed fluorescent compound T8 was 390 nm, and its energy value (AA) was 3.18 eV. ΔE_{ST} of the thermally activated delayed fluorescent compound T8 was 0.007 eV.

A thermally activated delayed fluorescent compound T9 manufactured by Luminescence Technology Corp. was used. The maximum peak wavelength of the emission spectrum at room temperature of the thermally activated delayed fluorescent compound T9 was 485 nm, and its energy value (EA) was 2.56 eV. The peak wavelength at the lowest energy side of the absorption spectrum at room temperature of the thermally activated delayed fluorescent compound T9 was 395 nm, and its energy value (AA) was 3.14 eV. ΔE_{ST} of the thermally activated delayed fluorescent compound T9 was 0.010 eV.

A thermally activated delayed fluorescent compound T10 manufactured by Luminescence Technology Corp. was used. The maximum peak wavelength of the emission spectrum at room temperature of the thermally activated delayed fluorescent compound T10 was 475 nm, and its energy value (EA) was 2.61 eV. The peak wavelength at the lowest energy side of the absorption spectrum at room temperature of the thermally activated delayed fluorescent compound T10 was 402 nm, and its energy value (AA) was 3.08 eV. ΔE_{ST} of the thermally activated delayed fluorescent compound T10 was 0.007 eV.

A thermally activated delayed fluorescent compound T11 manufactured by Luminescence Technology Corp. was used. The maximum peak wavelength of the emission spectrum at room temperature of the thermally activated delayed fluorescent compound T11 was 483 nm, and its energy value (EA) was 2.57 eV. The peak wavelength at the lowest energy side of the absorption spectrum at room temperature of the thermally activated delayed fluorescent compound T11 was 388 nm, and its energy value (AA) was 3.20 eV. ΔE_{ST} of the thermally activated delayed fluorescent compound T11 was 0.006 eV.

A thermally activated delayed fluorescent compound T12 manufactured by Luminescence Technology Corp. was used. The maximum peak wavelength of the emission spectrum at room temperature of the thermally activated delayed fluorescent compound T12 was 500 nm, and its energy value (EA) was 2.48 eV. The peak wavelength at the lowest energy side of the absorption spectrum at room temperature of the thermally activated delayed fluorescent compound T12 was 398 nm, and its energy value (AA) was 3.12 eV. ΔE_{ST} of the thermally activated delayed fluorescent compound T12 was 0.027 eV.

A compound E1 manufactured by Tokyo Chemical Industry Co., Ltd. was used. The maximum peak wavelength of the emission spectrum at room temperature of the compound E1 was 520 nm, and its energy value (EA) was 2.38 eV. The peak wavelength at the lowest energy side of the absorption spectrum at room temperature of the compound E1 was 511 nm, and its energy value (AA) was 2.43 eV. ΔE_{ST} of the compound E1 was 0.717 eV.

A compound B1 manufactured by Luminescence Technology Corp. was used. The maximum peak wavelength of the emission spectrum at room temperature of the compound B1 was 452 nm, and its energy value (EB) was 2.74 eV. The energy value of the half-value width of the maximum peak of the emission spectrum at room temperature of the compound B1 was 0.132 eV. The peak wavelength at the lowest energy side of the absorption spectrum at room temperature of the compound B1 was 439 nm, and its energy value (AB) was 2.82 eV. ΔE_{ST} of the compound B1 was 0.494 eV.

A compound B2 was synthesized with reference to a method described in International Publication WO2015/102118. The maximum peak wavelength of the emission spectrum at room temperature of the compound B2 was 440 nm, and its energy value (EB) was 2.82 eV. The energy value of the half-value width of the maximum peak of the emission spectrum at room temperature of the compound B2 was 0.127 eV. The peak wavelength at the lowest energy side of the absorption spectrum at room temperature of the compound B2 was 427 nm, and its energy value (AB) was 2.90 eV. ΔE_{ST} of the compound B2 was 0.471 eV.

A compound B3 was synthesized with reference to a method described in International Publication WO2015/102118. The maximum peak wavelength of the emission spectrum at room temperature of the compound B3 was 453 nm, and its energy value (EB) was 2.74 eV. The energy value of the half-value width of the maximum peak of the emission spectrum at room temperature of the compound B3 was 0.126 eV. The peak wavelength at the lowest energy side of the absorption spectrum at room temperature of the compound B3 was 439 nm, and its energy value (AB) was 2.82 eV. ΔE_{ST} of the compound B3 was 0.479 eV.

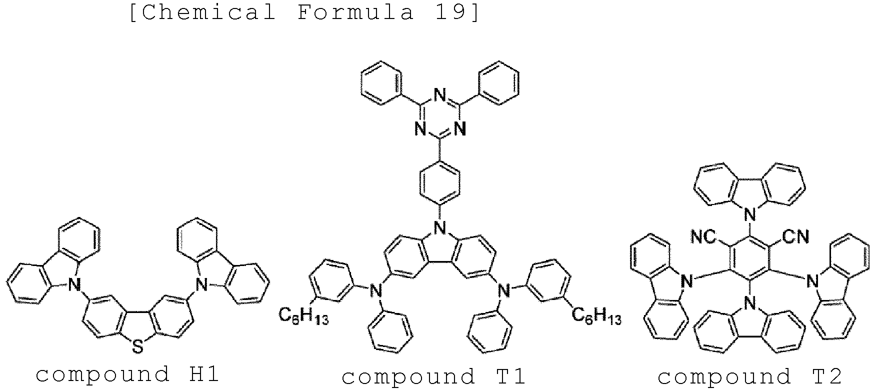

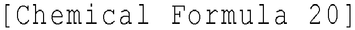

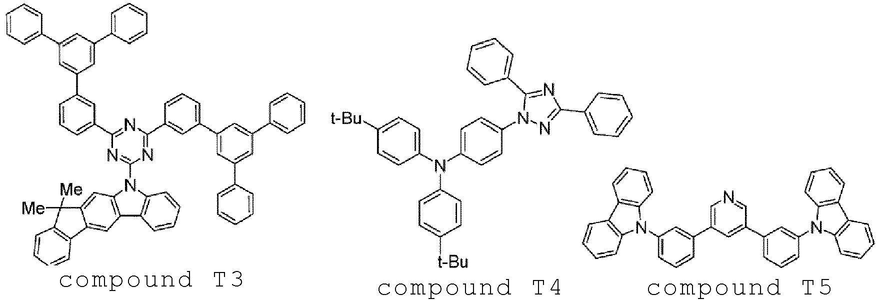

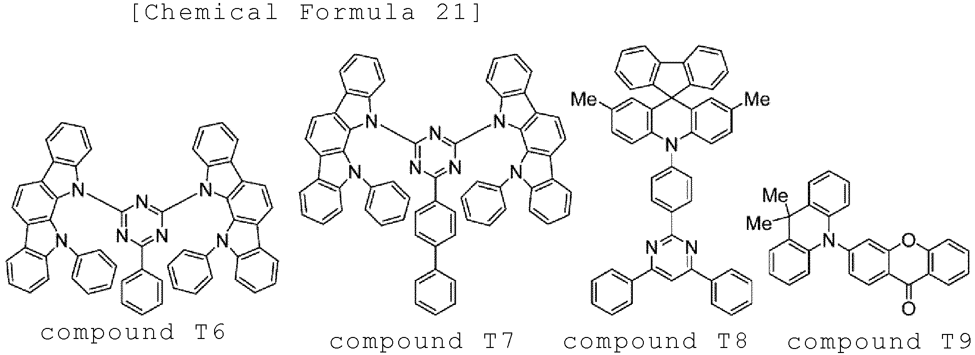

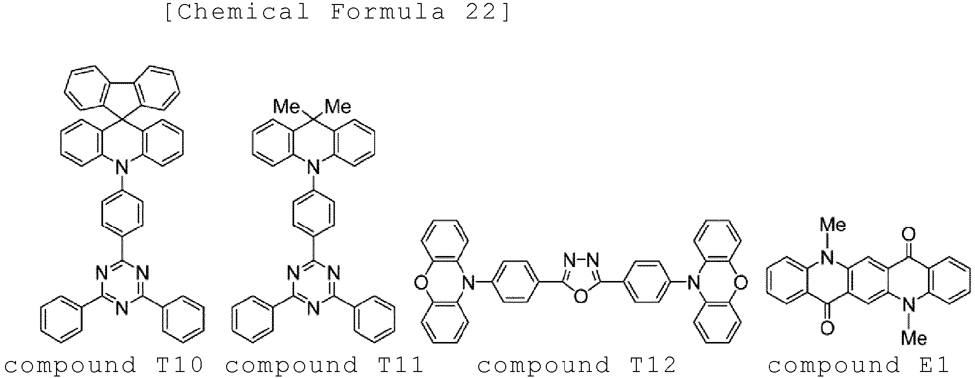

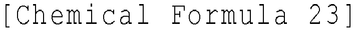

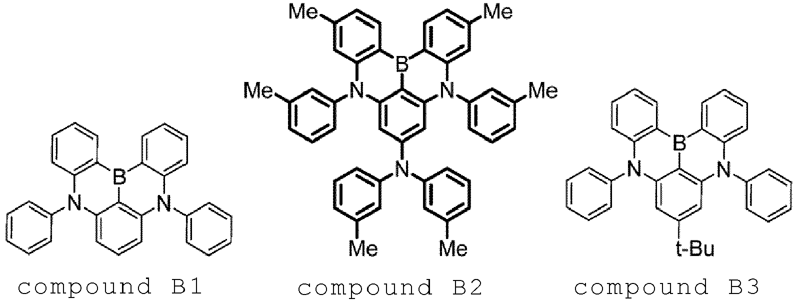

### <Example D1> Fabrication and evaluation of light emitting device D1

### (Formation of anode and hole injection layer)

An ITO film was attached with a thickness of 45 nm to a glass substrate by a sputtering method, to form an anode. On the anode, a hole injection material ND-3202 (manufactured by Nissan Chemical Corp.) was spin-coated, to form a film with a thickness of 35 nm. The substrate carrying the hole injection layer laminated thereon was heated on a hot plate at 50°C for 3 minutes, and further heated at 230°C for 15 minutes, under an air atmosphere, to form a hole injection layer.

### (Formation of hole transporting layer)

The polymer compound HTL-1 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer. The polymer compound HTL-1 is a polymer compound which is the same as the polymer compound HTL-13 described in examples of International Publication WO2018/062278.

### (Formation of light emitting layer)

The compound H1, the compound B1 and the thermally activated delayed fluorescent compound T2 (compound H1/compound Bl/thermally activated delayed fluorescent compound T2 = 95% by mass/4% by mass/1% by mass) were dissolved at a concentration of 2% by mass in toluene. The resultant toluene solution was spin-coated on the hole transporting layer, to form a film with a thickness of 60 nm, and the film was heated at 130°C for 10 minutes under a nitrogen gas atmosphere, to form a light emitting layer.

### (Formation of cathode)

The substrate carrying the light emitting layer formed thereon was placed in a vapor deposition machine and the internal pressure thereof was reduced to 1.0×10⁻⁴ Pa or less, then, as the cathode, sodium fluoride was vapor-deposited with a thickness of about 4 nm on the light emitting layer, then, aluminum was vapor-deposited with a thickness of about 80 nm on the sodium fluoride layer. After vapor deposition, the substrate carrying the cathode formed thereon was sealed with a glass substrate, to fabricate a light emitting device D1.

### (Evaluation of light emitting device)

Voltage was applied to the light emitting device D1, to observe EL light emission. The light emission efficiency [cd/A] at 400 cd/m² and the CIE chromaticity coordinate were measured. The results are shown in Table 1.

### <Examples D2 to D3 and Comparative Examples CD1 to CD3> Fabrication and evaluation of light emitting devices D2, D3 and CD1 to CD3

Light emitting devices D2, D3 and CD1 to CD3 were fabricated in the same manner as in Example D1, except that materials and composition ratios (% by mass) described in Table 1 were used instead of "the compound H1, the compound B1 and the thermally activated delayed fluorescent compound T2 (compound H1/compound Bl/thermally activated delayed fluorescent compound T2 = 95% by mass/4% by mass/1% by mass)" in (Formation of light emitting layer) of Example D1.

Voltage was applied to the light emitting devices D2, D3 and CD1 to CD3, to observe EL light emission. The light emission efficiency [cd/A] at 400 cd/m² and the CIE chromaticity coordinate were measured. The results are shown in Table 1.

The results of Examples D1 to D3 and Comparative Examples CD1 to CD3 are shown in Table 1. The relative values of the light emission efficiency of the light emitting devices D1 to D3, CD2 and CD3, when the light emission efficiency of the light emitting device CD1 is taken as 1.0, are shown.

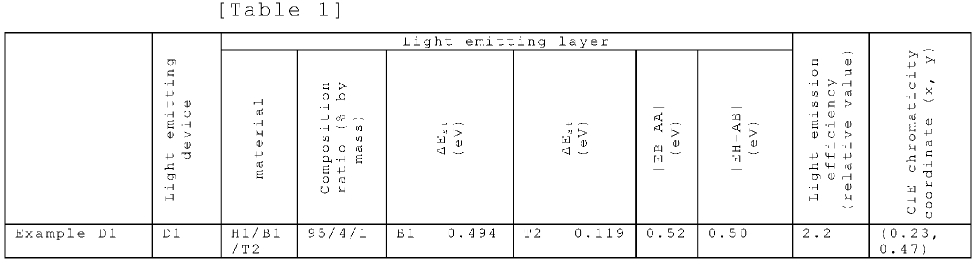

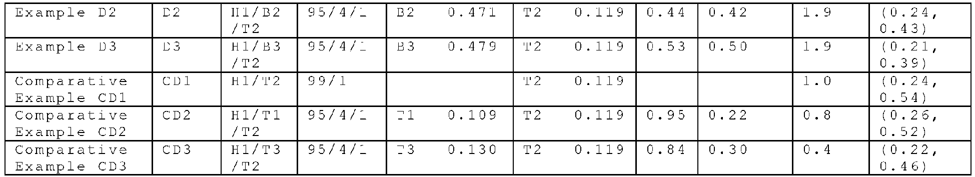

### <Examples D4 to D5 and Comparative Examples CD4 to CD5> Fabrication and evaluation of light emitting devices D4, D5, CD4 and CD5

Light emitting devices D4, D5, CD4 and CD5 were fabricated in the same manner as in Example D1, except that materials and composition ratios (% by mass) described in Table 2 were used instead of "the compound H1, the compound B1 and the thermally activated delayed fluorescent compound T2 (compound H1/compound Bl/thermally activated delayed fluorescent compound T2 = 95% by mass/4% by mass/1% by mass)" in (Formation of light emitting layer) of Example D1, and further, "the polymer compound HTL-2" was used instead of "the polymer compound HTL-1" in (Formation of hole transporting layer) of Example D1. The polymer compound HTL-2 is a polymer compound described in Polymer Example 1 of International Publication WO2014/102543.

Voltage was applied to the light emitting devices D4, D5, CD4 and CD5, to observe EL light emission. The light emission efficiency [cd/A] at 100 cd/m² and the CIE chromaticity coordinate were measured. The results are shown in Table 2.

The results of Examples D4 to D5 and Comparative Examples CD4 to CD5 are shown in Table 2. The relative values of the light emission efficiency of the light emitting devices D4, D5 and CD5, when the light emission efficiency of the light emitting device CD4 is taken as 1.0, are shown.

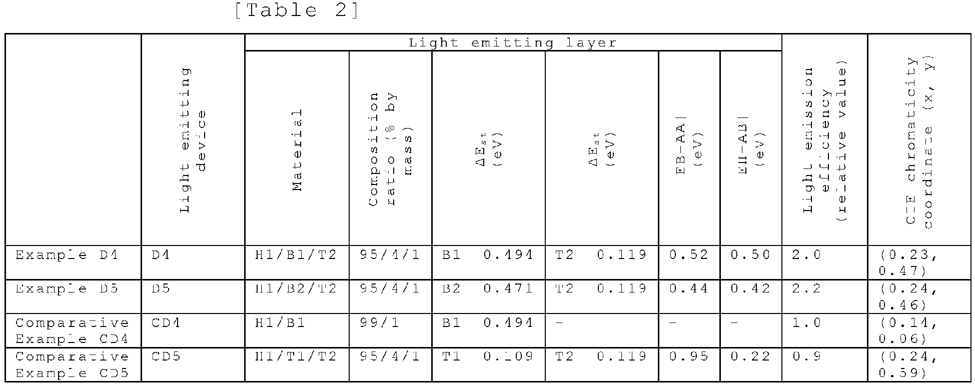

### <Examples D6 to D10 and Comparative Examples CD6 to CD8> Fabrication and evaluation of light emitting devices D6 to D10 and CD6 to CD8

Light emitting devices D6 to D10 and CD6 to CD8 were fabricated in the same manner as in Example D1, except that materials and composition ratios (% by mass) described in Table 3 were used instead of "the compound H1, the compound B1 and the thermally activated delayed fluorescent compound T2 (compound H1/compound Bl/thermally activated delayed fluorescent compound T2 = 95% by mass/4% by mass/1% by mass)" in (Formation of light emitting layer) of Example D1, and further, "the polymer compound HTL-3" was used instead of "the polymer compound HTL-1" in (Formation of hole transporting layer) of Example D1. The polymer compound HTL-3 is a polymer compound which is the same as the polymer compound HTL-2 described in examples of International Publication WO2018/062276.

Voltage was applied to the light emitting devices D6 to D10 and CD6 to CD8, to observe EL light emission. The light emission efficiency [cd/A] at 100 cd/m² and the CIE chromaticity coordinate were measured. The results are shown in Table 3.

The results of Examples D6 to D10 and Comparative Examples CD6 to CD8 are shown in Table 3. The relative values of the light emission efficiency of the light emitting devices D6 to D10, CD6 and CD7, when the light emission efficiency of the light emitting device CD8 is taken as 1.0, are shown.

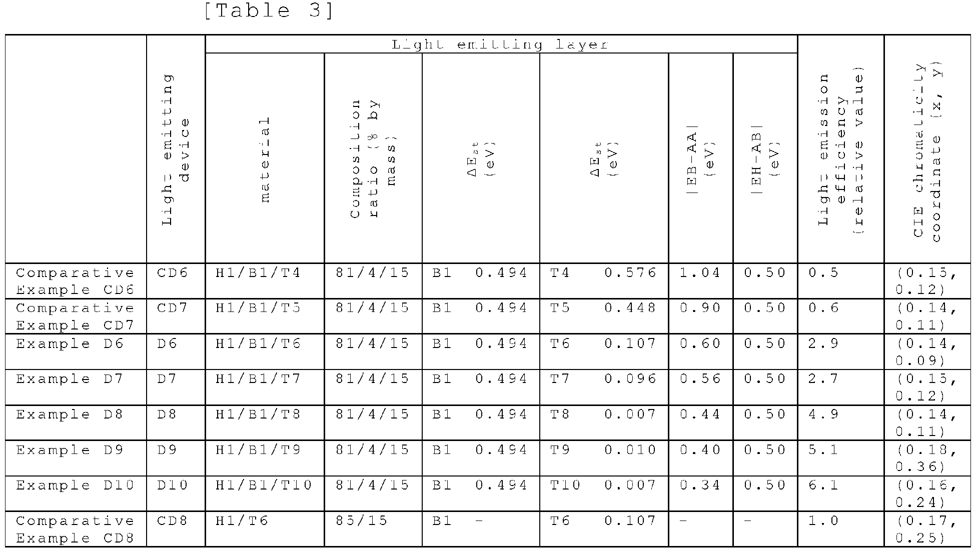

### <Example D11 to D14 and Comparative Example CD9>

Fabrication and evaluation of light emitting devices D11 to D14 and CD9

Light emitting devices D11 to D14 and CD9 were fabricated in the same manner as in Example D1, except that materials and composition ratios (% by mass) described in Table 3 were used instead of "the compound H1, the compound B1 and the thermally activated delayed fluorescent compound T2 (compound H1/compound Bl/thermally activated delayed fluorescent compound T2 = 95% by mass/4% by mass/1% by mass)" in (Formation of light emitting layer) of Example D1, and further, "the polymer compound HTL-3" was used instead of "the polymer compound HTL-1" in (Formation of hole transporting layer) of Example D1. The polymer compound HTL-3 is a polymer compound which is the same as the polymer compound HTL-2 described in examples of International Publication WO2018/062276.

Voltage was applied to the light emitting devices D11 to D14 and CD9, to observe EL light emission. The light emission efficiency [cd/A] at 50 cd/m² and the CIE chromaticity coordinate were measured. The results are shown in Table 4.

The results of Examples D11 to D14 and Comparative Example CD9 are shown in Table 4. The relative values of the light emission efficiency of the light emitting devices D11 to D14, when the light emission efficiency of the light emitting device CD9 is taken as 1.0, are shown.

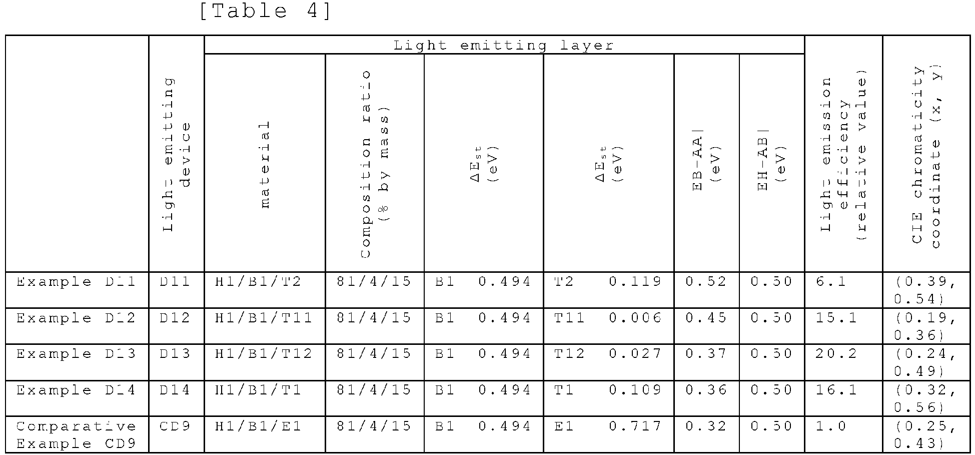

### Industrial Applicability

The composition of the present invention is useful for producing a light emitting device excellent in light emission efficiency.

## Claims

1. A light emitting device comprising
an anode,
a cathode, and
an organic layer disposed between said anode and said cathode and containing a composition for light emitting device, wherein
said composition for light emitting device contains
a thermally activated delayed fluorescent compound (A), and
a compound (B) having a condensed hetero ring skeleton (b) containing a boron atom and at least one selected from the group consisting of an oxygen atom, a sulfur atom, a selenium atom, an sp³ carbon atom and a nitrogen atom in the ring,
said thermally activated delayed fluorescent compound (A) is a compound not having said condensed hetero ring skeleton (b),
the absolute value (|EB-AA|) of a difference between the energy value (EB) of the maximum peak of the emission spectrum at 25°C of said compound (B) and the energy value (AA) of a peak at the lowest energy side of the absorption spectrum at 25°C of said compound (A) is 0.60 eV or less,
the absolute value ΔE_{ST}(A) of a difference between the energy level of the lowest triplet excited state and the energy level of the lowest singlet excited state of said compound (A) is 0.50 eV or less, and
the absolute value ΔE_{ST}(B) of a difference between the energy level of the lowest triplet excited state and the energy level of the lowest singlet excited state of said compound (B) is 0.50 eV or less.

2. The light emitting device according to Claim 1, wherein said ΔE_{ST}(B) is larger than said ΔE_{ST}(A).

3. The light emitting device according to Claim 1 or 2, wherein said condensed hetero ring skeleton (b) contains a boron atom and at least one selected from the group consisting of an oxygen atom, a sulfur atom and a nitrogen atom in the ring.

4. The light emitting device according to any one of Claims 1 to 3, wherein said compound (B) is a compound represented by the formula (1-1), a compound represented by the formula (1-2) or a compound represented by the formula (1-3):
[Chemical Formula 1] 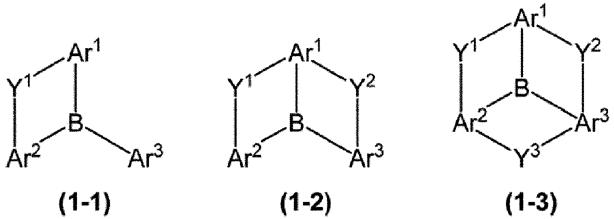 wherein,
Ar¹, Ar² and Ar³ each independently represent an aromatic hydrocarbon group or a hetero ring group, and these groups optionally have a substituent, when a plurality of the substituents are present, they may be the same or different and may be combined together to form a ring together with atoms to which they are attached,
Y¹ represents an oxygen atom, a sulfur atom, a selenium atom, a group represented by -N(Ry)-, an alkylene group or a cycloalkylene group, and these groups optionally have a substituent, when a plurality of the substituents are present, they may be the same or different and may be combined together to form a ring together with atoms to which they are attached,
Y² and Y³ each independently represent a single bond, an oxygen atom, a sulfur atom, a selenium atom, a group represented by -N(Ry)-, an alkylene group or a cycloalkylene group, and these groups optionally have a substituent, when a plurality of the substituents are present, they may be the same or different and may be combined together to form a ring together with atoms to which they are attached, Ry represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of Ry are present, they may be the same or different, Ry may be bonded directly or via a connecting group to Ar¹, Ar² or Ar³.

5. The light emitting device according to Claim 4, wherein said Y¹, said Y² and said Y³ are each an oxygen atom, a sulfur atom or a group represented by -N(Ry)-.

6. The light emitting device according to any one of Claims 1 to 5, wherein said compound (A) is a compound represented by the formula (T-1):
[Chemical Formula 2] 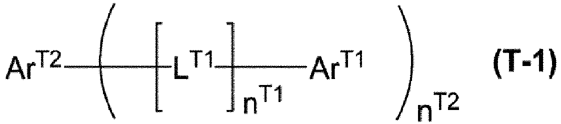 wherein,
n^{T1} represents an integer of 0 or more, when a plurality of n^{T1} are present, they may be the same or different,
n^{T2} represents an integer of 1 or more, n^{T2} is 2, when Ar^{T2} is a group represented by -C(=O)-, a group represented by -S(=O)- or a group represented by -S(=O)₂-,
Ar^{T1} represents a substituted amino group or a monovalent hetero ring group, and these groups optionally have a substituent, when a plurality of the substituents are present, they may be the same or different and may be combined together to form a ring together with atoms to which they are attached, when a plurality of Ar^{T1} are present, they may be the same or different,
the monovalent hetero ring group represented by Ar^{T1} is a monovalent hetero ring group containing a nitrogen atom not forming a double bond in the ring and not containing a group represented by =N-, a group represented by -C(=O)-, a group represented by -S(=O)- and a group represented by -S(=O)₂- in the ring,
L^{T1} represents an alkylene group, a cycloalkylene group, an arylene group, a divalent hetero ring group, an oxygen atom or a sulfur atom, and these groups optionally have a substituent, when a plurality of the substituents are present, they may be the same or different and may be combined together to form a ring together with atoms to which they are attached, when a plurality of L^{T1} are present, they may be the same or different,
Ar^{T2} is a group represented by -C(=O)-, a group represented by -S(=O)-, a group represented by - S(=O)₂-, an aromatic hydrocarbon group having an electron-attracting group, an aromatic hydrocarbon group containing a group represented by -C(=O)- in the ring or a hetero ring group containing at least one group selected from the group consisting of a group represented by =N-, a group represented by - C(=O)-, a group represented by -S(=O)- and a group represented by -S(=O)₂- in the ring, and these groups optionally have a substituent, when a plurality of the substituents are present, they may be the same or different and may be combined together to form a ring together with atoms to which they are attached.

7. The light emitting device according to any one of Claims 1 to 6, wherein said composition for light emitting device further contains a host material.

8. The light emitting device according to Claim 7, wherein said host material contains a compound represented by the formula (H-1): 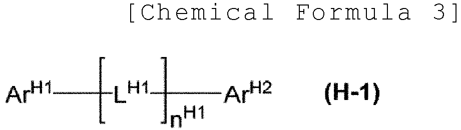 wherein,
Ar^{H1} and Ar^{H2} each independently represent an aryl group, a monovalent hetero ring group or a substituted amino group, and these groups optionally have a substituent, when a plurality of the substituents are present, they may be the same or different and may be combined together to form a ring together with atoms to which they are attached,
n^{H1} represents an integer of 0 or more,
L^{H1} represents an arylene group, a divalent hetero ring group, an alkylene group or a cycloalkylene group, and these groups optionally have a substituent, when a plurality of the substituents are present, they may be the same or different and may be combined together to form a ring together with atoms to which they are attached, when a plurality of L^{H1} are present, they may be the same or different.

9. The light emitting device according to Claim 7 or 8, wherein the absolute value (|EH-AB|) of a difference between the energy value (EH) of the maximum peak of the emission spectrum at 25°C of said host material and the energy value (AB) of a peak at the lowest energy side of the absorption spectrum at 25°C of said compound (B) is 0.60 eV or less.

10. The light emitting device according to any one of Claims 1 to 9, wherein said composition for light emitting device further contains at least one selected from the group consisting of a hole transporting material, a hole injection material, an electron transporting material, an electron injection material, a light emitting material, an antioxidant and a solvent.

11. A composition for light emitting device comprising
a thermally activated delayed fluorescent compound (A) and
a compound (B) having a condensed hetero ring skeleton (b) containing a boron atom and at least one selected from the group consisting of an oxygen atom, a sulfur atom, a selenium atom, an sp³ carbon atom and a nitrogen atom in the ring, wherein
said thermally activated delayed fluorescent compound (A) is a compound not having said condensed hetero ring skeleton (b),
the absolute value (|EB-AA|) of a difference between the energy value (EB) of the maximum peak of the emission spectrum at 25°C of said compound (B) and the energy value (AA) of a peak at the lowest energy side of the absorption spectrum at 25°C of said compound (A) is 0.60 eV or less,
the absolute value ΔE_{ST}(A) of a difference between the energy level of the lowest triplet excited state and the energy level of the lowest singlet excited state of said compound (A) is 0.50 eV or less, and
the absolute value ΔE_{ST}(B) of a difference between the energy level of the lowest triplet excited state and the energy level of the lowest singlet excited state of said compound (B) is 0.50 eV or less.

12. The composition for light emitting device according to Claim 11, further comprising a host material.

13. The composition for light emitting device according to Claim 12, wherein said host material contains a compound represented by the formula (H-1) : 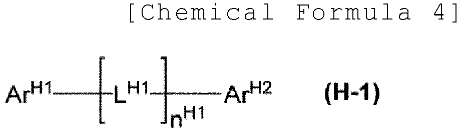 wherein,
Ar^{H1} and Ar^{H2} each independently represent an aryl group, a monovalent hetero ring group or a substituted amino group, and these groups optionally have a substituent, when a plurality of the substituents are present, they may be the same or different and may be combined together to form a ring together with atoms to which they are attached,
n^{H1} represents an integer of 0 or more,
L^{H1} represents an arylene group, a divalent hetero ring group, an alkylene group or a cycloalkylene group, and these groups optionally have a substituent, when a plurality of the substituents are present, they may be the same or different and may be combined together to form a ring together with atoms to which they are attached, when a plurality of L^{H1} are present, they may be the same or different.

14. The composition for light emitting device according to Claim 12 or 13, wherein the absolute value (|EH-AB|) of a difference between the energy value (EH) of the maximum peak of the emission spectrum at 25°C of said host material and the energy value (AB) of a peak at the lowest energy side of the absorption spectrum at 25°C of said compound (B) is 0.60 eV or less.

15. The composition for light emitting device according to any one of Claims 11 to 14, wherein said composition for light emitting device further contains at least one selected from the group consisting of a hole transporting material, a hole injection material, an electron transporting material, an electron injection material, a light emitting material, an antioxidant and a solvent.

16. A method for producing a composition for light emitting device, comprising
a preparation step of preparing a thermally activated delayed fluorescent compound (A) in which the absolute value ΔE_{ST}(A) of a difference between the energy level of the lowest triplet excited state and the energy level of the lowest singlet excited state is 0.50 eV or less,
a sorting step of sorting a compound (B) which is a compound having a condensed hetero ring skeleton (b) containing a boron atom and at least one selected from the group consisting of an oxygen atom, a sulfur atom, a selenium atom, an sp³ carbon atom and a nitrogen atom in the ring and in which the absolute value ΔE_{ST}(B) of a difference between the energy level of the lowest triplet excited state and the energy level of the lowest singlet excited state is 0.50 eV or less and the energy value (EB) of the maximum peak of the emission spectrum at 25°C shows a value with which the absolute value (|EB-AA|) of a difference from the energy value (AA) of a peak at the lowest energy side of the absorption spectrum at 25°C of said compound (A) is 0.60 eV or less, and
a production step of mixing the compound (A) prepared in said preparation step and the compound (B) sorted in said sorting step to obtain a composition for light emitting device, wherein
said thermally activated delayed fluorescent compound (A) is a compound not having said condensed hetero ring skeleton (b).

17. The production method according to Claim 16, wherein said sorting step includes a step of determining the energy value (EB) of the maximum peak of the emission spectrum at 25°C of said compound (B) and the energy value (AA) of a peak at the lowest energy side of the absorption spectrum at 25°C of said compound (A) and calculating the absolute value (|EB-AA|) of a difference thereof.

18. The production method according to Claim 16 or 17, wherein said production step is a step of mixing said compound (A) prepared in said preparation step, said compound (B) sorted in said sorting step, and a host material.

19. The production method according to Claim 18, wherein
said sorting step further includes a step of sorting said compound (B) such that the absolute value (|EH-AB|) of a difference between the energy value (EH) of the maximum peak of the emission spectrum at 25°C of said host material and the energy value (AB) of a peak at the lowest energy side of the absorption spectrum at 25°C of said compound (B) is 0.60 eV or less.

20. The production method according to Claim 16 or 17, further comprising a host material sorting step of sorting the host material such that the absolute value (|EH-AB|) of a difference between the energy value (EH) of the maximum peak of the emission spectrum at 25°C of the host material and the energy value (AB) of a peak at the lowest energy side of the absorption spectrum at 25°C of said compound (B) sorted in said sorting step is 0.60 eV or less, wherein
said production step is a step of mixing said compound (A) prepared in said preparation step, said compound (B) sorted in said sorting step, and said host material sorted in said host material sorting step.

21. The production method according to any one of Claims 18 to 20, wherein said host material contains a compound represented by the formula (H-1): 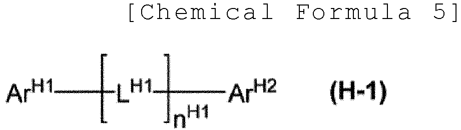 wherein,
Ar^{H1} and Ar^{H2} each independently represent an aryl group, a monovalent hetero ring group or a substituted amino group, and these groups optionally have a substituent, when a plurality of the substituents are present, they may be the same or different and may be combined together to form a ring together with atoms to which they are attached,
n^{H1} represents an integer of 0 or more,
L^{H1} represents an arylene group, a divalent hetero ring group, an alkylene group or a cycloalkylene group, and these groups optionally have a substituent, when a plurality of the substituents are present, they may be the same or different and may be combined together to form a ring together with atoms to which they are attached, when a plurality of L^{H1} are present, they may be the same or different.

22. A method for producing a composition for light emitting device, comprising
a preparation step of preparing a compound (B) in which the absolute value ΔE_{ST}(B) of a difference between the energy level of the lowest triplet excited state and the energy level of the lowest singlet excited state is 0.50 eV or less, and having a condensed hetero ring skeleton (b) containing a boron atom and at least one selected from the group consisting of an oxygen atom, a sulfur atom, a selenium atom, an sp³ carbon atom and a nitrogen atom in the ring,
a sorting step of sorting a thermally activated delayed fluorescent compound (A) in which the absolute value ΔE_{ST}(A) of a difference between the energy level of the lowest triplet excited state and the energy level of the lowest singlet excited state is 0.50 eV or less, and, the energy value (AA) of a peak at the lowest energy side of the absorption spectrum at 25°C shows a value with which the absolute value (|EB-AA|) of a difference from the energy value (EB) of the maximum peak of the emission spectrum at 25°C of said compound (B) is 0.60 eV or less, and
a production step of mixing the compound (B) prepared in said preparation step and said compound (A) sorted in said sorting step to obtain a composition for light emitting device, wherein
said thermally activated delayed fluorescent compound (A) is a compound not having said condensed hetero ring skeleton (b).

23. The production method according to Claim 22, wherein said sorting step includes a step of determining the energy value (EB) of the maximum peak of the emission spectrum at 25°C of said compound (B) and the energy value (AA) of a peak at the lowest energy side of the absorption spectrum at 25°C of said compound (A) and calculating the absolute value (|EB-AA|) of a difference thereof.

24. The production method according to Claims 22 or 23, wherein said production step is a step of mixing said compound (B) prepared in said preparation step, said compound (A) sorted in said sorting step, and a host material.

25. The production method according to Claim 24, further comprising a host material preparation step of preparing a host material, wherein
said preparation step is a step of preparing a compound (B) in which the absolute value (|EH-AB|) of a difference between the energy value (EH) of the maximum peak of the emission spectrum at 25°C of said host material and the energy value (AB) of a peak at the lowest energy side of the absorption spectrum at 25°C of said compound (B) is 0.60 eV or less.

26. The production method according to Claim 22 or 23, further comprising a host material sorting step of sorting a host material such that the absolute value (|EH-AB|) of a difference between the energy value (EH) of the maximum peak of the emission spectrum at 25°C of the host material and the energy value (AB) of a peak at the lowest energy side of the absorption spectrum at 25°C of said compound (B) prepared in said preparation step is 0.60 eV or less, wherein
said production step is a step of mixing said compound (B) prepared in said preparation step, said compound (A) sorted in said sorting step, and said host material sorted in said host material sorting step.

27. The production method according to any one of Claims 24 to 26, wherein said host material contains a compound represented by the formula (H-1) : 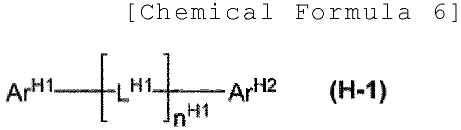 wherein,
Ar^{H1} and Ar^{H2} each independently represent an aryl group, a monovalent hetero ring group or a substituted amino group, and these groups optionally have a substituent, when a plurality of the substituents are present, they may be the same or different and may be combined together to form a ring together with atoms to which they are attached,
n^{H1} represents an integer of 0 or more,
L^{H1} represents an arylene group, a divalent hetero ring group, an alkylene group or a cycloalkylene group, and these groups optionally have a substituent, when a plurality of the substituents are present, they may be the same or different and may be combined together to form a ring together with atoms to which they are attached, when a plurality of L^{H1} are present, they may be the same or different.

28. A method for producing a light emitting device having an anode, a cathode, and an organic layer disposed between said anode and said cathode, comprising
a step of producing a composition for light emitting device by the production method as described in any one of Claims 16 to 27, and a step of forming said organic layer using said composition for light emitting device produced in said step.
